Europäisches Patentamt

European Patent Office

Office européen des brevets

(11) Numéro de publication: **0 548 175 B1**

(12) **FASCICULE DE BREVET EUROPEEN**

(45) Date de publication du fascicule du brevet: 06.09.95

(51) Int. Cl.⁶: **C12N 15/76**, C12N 15/31, C07K 14/00, C12N 1/21, //C12N1:21,(C12R1/465,1:32)

(21) Numéro de dépôt: **91916291.7**

(22) Date de dépôt: **03.09.91**

(86) Numéro de dépôt internationale :
**PCT/FR91/00701**

(87) Numéro de publication internationale :
**WO 92/04452 (19.03.92 92/07)**

(54) **SEQUENCE NUCLEOTIDIQUE REGULATRICE DE L'INITIATION DE TRANSCRIPTION.**

(30) Priorité: **10.09.90 FR 9011186**

(43) Date de publication de la demande:
**30.06.93 Bulletin 93/26**

(45) Mention de la délivrance du brevet:
**06.09.95 Bulletin 95/36**

(84) Etats contractants désignés:
**AT BE CH DE DK ES FR GB GR IT LI LU NL SE**

(56) Documents cités:
**EP-A- 0 179 449**
**EP-A- 0 352 707**

**Journal of Antibiotics, volume XL, no. 1, octobre 1987, (Tokyo, JP) T. Manome et al.: "Cloning of DNA fragments containing streptomyces promoter activity", pages 1440-1447, voir l'article en entier**

(73) Titulaire: **INSTITUT PASTEUR**
**25-28, rue du Docteur Roux**
**F-75724 Paris Cédex 15 (FR)**

(72) Inventeur: **MAZODIER, Philippe**
**34, rue des Sablons**
**F-92140 Clamart (FR)**
Inventeur: **GUGLIELMI, Gérard**
**37-39, rue Collange**
**F-92300 Levallois-Perret (FR)**

(74) Mandataire: **Desaix, Anne et al**
**Ernest Gutmann - Yves Plasseraud S.A.**
**3, rue Chauveau-Lagarde**
**F-75008 Paris (FR)**

EP 0 548 175 B1

Gene, volume 51, nos. 2-3, 1987, (Amsterdam, NL) M.J. Buttner et al.:"Two promoters from the streptomyces plasmid pIJ101 and their expression in escherichia coli", pages 179-186, voir l'article en entier

Journal of General Microbiology, volume 135, partie 4, avril 1989, (GB) P.N. Baird et al.: "Cloning and sequence analysis of the 10 kDA antigen gene of mycobacterium tuberculosis", pages 931-939, voir l'article en entier

2

**Description**

L'invention concerne une séquence nucléotidique, régulatrice de l'initiation de transcription et son utilisation dans la production, par voie recombinante, de polypeptides.

Le problème technique qui se posait lors de l'élaboration de la présente invention était d'identifier un promoteur fort, et éventuellement thermoinductible, utilisable chez un grand nombre de microorganismes, et en particulier chez les Actinomycètes.

Les Actinomycètes constituent un ordre bactérien d'une importance économique et médicale considérable, rappelons seulement que les Actinomycètes comportent notamment les genres Streptomyces et Mycobacterium.

Les Streptomyces sont utilisés pour la production d'environ 70 % des antibiotiques aujourd'hui commercialisés ; en outre, si les méfaits de Mycobacterium tuberculosis et Mycobacterium leprae ne sont plus à décrire, il existe un grand intérêt pour l'expression d'antigène hétérologue dans la souche de M. bovis BCG afin d'obtenir une souche vaccinale vivante polyvalente.

La génétique de ces bactéries a été peu étudiée au niveau moléculaire, la régulation de l'expression génétique semble toutefois différente de celle qui a été décrite pour des bactéries plus étudiées comme Escherichia coli ou Bacillus subtilis.

En particulier, les Actinomycètes, dont l'ADN est généralement riche en G + C, reconnaissent peu et mal les séquences "promoteurs" caractérisés chez Escherichia coli ou Bacillus subtilis.

Baird et al (J. Gen. Microbiol. 1989, 135, 931-939) ont étudié les gènes codant pour des protéines de choc thermique chez Mycobacterium et ont postulé que deux séquences TTGAG et TCTCATGT, se trouvant en amont de la séquence codant pour la protéine de choc thermique de 10 kDa chez Mycobacterium tuberculosis, constituent les régions -35 et -10 du promoteur.

En effet, ces deux séquences présentent une homologie élevée avec la séquence du promoteur consensus chez E. coli. En outre, des séquences se trouvant en amont des gènes codant pour d'autres protéines de choc thermique chez différentes espèces de Mycobacterium (par exemple, la protéine de 65 kDa de Mycobacterium leprae ou celle de 64 kDa chez M. bovis) renferment également un couple de séquences du même type, c'est-à-dire : TTGCCG et TTTCAT, ou TTGCCG et CTTCAT, et qui présentent donc une homologie élevée avec le promoteur d'E. coli et avec les séquences "-35 et -10" du Mycobacterium tuberculosis 10 kDa.

D'après cet article et celui de Thole et al. (Infection and Immunity, 55 1987, 1466-1475), les promoteurs responsables de la transcription des gènes de protéines de choc thermique chez Mycobacterium renferment, en tant que séquences -35 et -10, ce type de couple de séquences. Ces séquences seront appelées dans ce qui suit "séquences -10 et -35 du type E. coli". Ceci étant, les auteurs n'avaient pas confirmé cette hypothèse par cartographie et l'identité des promoteurs reste par conséquent incertaine.

D'une manière surprenante, les présents inventeurs ont constaté que la séquence -10 et -35 du type E. coli est également présente chez Streptomyces, mais ne joue pas le rôle d'initiation de transcription des protéines de choc thermique chez des bactéries de ce genre.

Il est à noter que Baird et Thole, dans les articles cités ci-dessus, avaient en outre constaté la présence, dans des séquences en amont des gènes codant pour les protéines de choc thermique chez Mycobacterium, d'un motif palindromique comportant la séquence GCACTC 9N GAGTGC. Toutefois, le rôle précis de ce motif n'avait pas été identifié. Thole postule que ce motif est impliqué soit dans la terminaison de transcription d'un opéron se trouvant en amont du gène codant pour la protéine de choc thermique, soit dans la régulation de traduction d'un messager polycistronique.

Dans le but de cloner un promoteur fort (et éventuellement thermoinductible), utilisable chez un grand nombre d'Actinomycètes, les inventeurs ont étudié la réponse au choc thermique chez les Streptomyces et ont cloné une protéine fortement exprimée afin de caractériser son promoteur. En effet, le phénomène de réponse à un choc thermique par synthèse de novo de protéines est un phénomène universel ; on peut donc prévoir que sa régulation sera semblable chez divers Actinomycètes et notamment que les promoteurs auront des séquences consensuelles permettant leur utilisation dans un grand nombre de souches.

Les inventeurs ont ainsi identifié et caractérisé deux promoteurs fonctionnels chez les Streptomyces et, en outre, suite à l'observation que chacun de ces promoteurs renfermait le motif GCACTC 9N GAGTGC ont pu identifier la fonction dudit motif.

L'invention concerne une séquence nucléotidique recombinante caractérisée en ce qu'elle comprend :

- une séquence régulatrice de l'initiation de transcription, cette séquence régulatrice comportant un promoteur en association avec le motif GCACTC 9N GAGTGC dans lequel "N" signifie l'une quelconque des 4 bases : thymine, guanine, adénine et cytosine ;

- une séquence codant pour un polypeptide dit "polypeptide hétérologue", autre que celle naturellement associée avec ledit promoteur ; ladite séquence codante étant positionnée en aval de ladite séquence régulatrice de l'initiation de transcription, à un site qui, dans des conditions appropriées, permettrait l'expression du polypeptide sous le contrôle dudit promoteur.

La séquence nucléotidique recombinante de l'invention est apte à permettre l'expression d'un gène hétérologue dans une cellule la contenant.

La séquence régulatrice de l'initiation de transcription, qui fait partie de la séquence nucléotidique recombinante, est composée d'un promoteur en association avec le motif GCACTC 9H GAGTGC. Dans ce contexte, "en association" veut dire que le motif GCACTC 9N GAGTGC peut être distinct du promoteur ou qu'il peut être compris, au moins en partie, dans le promoteur. Cette dernière possibilité englobe à la fois la situation où le motif chevauche la séquence promotrice et la situation où le motif constitue une partie intégrale du promoteur.

Des promoteurs associés avec le motif GCACTC 9N GAGTGC, qui sont particulièrement préférés selon l'invention, sont les promoteurs présents chez les Actinomycètes et plus particulièrement ceux qui, dans le génome de la bactérie, sont normalement associés à des protéines de choc thermique. Comme exemples de ce type de promoteurs, on peut citer les promoteurs des protéines de choc thermique de 18 kDa (P1) et de 56 kDa (P2) chez <u>Streptomyces albus</u> identifiés dans le cadre de l'invention par les inventeurs :

<u>P1</u> correspondant à l'une des séquences :

**CATTGGCACTCCGCTTGACCGAGTGCTAATCGCGGTCATAGTCTCAGCTCTG**

**5'** **3'**

**ou**

**GCATTGGCACTCCGCTTGACCGAGTGCTAATCGCGGTCATAGTCTCAGCTCTG**

**5'** **3'**

<u>P2</u> correspondant à l'une des séquences :

**GGAGGCCCCTAGCGCCTGCACTCTCCTACCCCGAGTGCTATTATTGGCGTTA**

**5'** **3'**

**ou**

**GGAGGCCCCTAGCGCCTGCACTCTCCTACCCCGAGTGCTAATTATTGGCGTTA**

**5'** **3'**

Chacun de ces promoteurs peut être raccourci de 4 ou 5 bases maximum du côté 5', sans nuire à son activité. D'autres types de promoteurs sont ceux des protéines de choc thermique de <u>Mycobacterium tuberculosis</u> 10 kDa et 65 kDa, de <u>M. bovis</u> 64 kDa et de <u>M. leprae</u> 65 kDa.

L'association du motif GCACTC 9N GAGTGC avec le promoteur confère un caractère thermoinductible au promoteur. Il est probable que ce motif soit un opérateur et constitue le site de fixation d'un répresseur, empêchant ainsi la RNA polymérase de se fixer sur les séquences -10 et -35 du promoteur.

Dans le cas où le motif est distinct du promoteur, il est de préférence en amont du promoteur, par exemple d'environ 150 à 200 bases.

La séquence recombinante de l'invention comporte, outre la séquence régulatrice d'initiation de transcription, une séquence codant pour un polypeptide dit "polypeptide hétérologue", autre que celle naturellement associée audit promoteur. Le promoteur est ainsi dans un environnement génétique immédiat différent de celui qui l'entoure dans le génome dont il est issu. Comme exemples de types de polypeptides hétérologues, on peut citer des antigènes neutralisants utilisables dans la production de vaccins vivants recombinants ou des polypeptides conférant une résistance à un antibiotique, des enzymes, etc...

Dans la séquence nucléotidique de l'invention, la séquence codante est positionnée en aval de ladite séquence régulatrice. Bien entendu, leurs positions relatives sont telles que l'expression de la séquence codante a lieu sous le contrôle du promoteur.

L'invention concerne en outre un vecteur d'expression contenant la séquence nucléotidique de l'invention, par exemple un plasmide.

L'invention vise également une cellule transformée par ce vecteur d'expression, ladite cellule étant capable de reconnaître le promoteur utilisé dans la séquence régulatrice d'initiation de transcription. Les cellules transformées sont de préférence des cellules procaryotes, et plus particulièrement, les procaryotes appartenant à l'ordre des Actinomycètes, par exemple Streptomyces ou Mycobacterium.

L'invention concerne en outre un procédé pour la production d'un polypeptide caractérisé en ce qu'il comprend les étapes suivantes :

- transformation d'une cellule par un vecteur d'expression, selon l'invention, dans des conditions permettant l'expression dudit polypeptide, ladite cellule étant capable de reconnaître ledit promoteur ;
- récupération du polypeptide exprimé.

Les conditions permettant l'expression du polypeptide sont celles connues dans l'art, et dans le cas présent incluent, de préférence, l'application d'un choc thermique, ce qui a pour effet d'induire l'expression. Le choc thermique peut être une augmentation de la température pour atteindre environ 37°C à 45°C ou en particulier 40°C à 45°c, par exemple 37°C ou 41°C chez Streptomyces, 42°C à 45°C pour Mycobacterium.

Il est intéressant de noter que l'utilisation des promoteurs P1 et P2 de l'invention résulte en une expression maintenue de la protéine hétérologue à haute température, par exemple chez Streptomyces entre 37 et 41°C.

Un autre aspect de l'invention concerne la possibilité de transformer un promoteur en un promoteur thermoinductible par son association avec le motif GCACTC 9N GAGTGC. Plus particulièrement, cet aspect de l'invention concerne un procédé pour conférer un caractère thermoinductible à un promoteur, caractérisé par la juxtaposition d'une part, d'une séquence comprenant le motif GCACTC 9N GAGTGC et, d'autre part, du promoteur, la séquence comprenant le motif GCACTC 9N GAGTGC étant positionnée en amont du promoteur, ou par l'insertion de la séquence comprenant le motif GCACTC 9N GAGTGC à un site qui est, au moins en partie, au sein du promoteur, ce dernier site étant choisi de manière à ce que la simple insertion dudit motif ne perturbe pas l'activité du promoteur.

Bien entendu, la position précise que doit avoir le motif GCACTC 9N GAGTGC par rapport au promoteur pour pouvoir conférer le caractère thermoinductible peut varier selon le promoteur utilisé. Ceci peut être vérifié en détectant, lors de l'application d'un choc thermique, l'expression d'un gène hétérologue facilement décelable, par exemple un gène marqueur comme LacZ, dans une cellule transformée par la construction sous test. Le positionnement du motif GCACTC 9N GAGTGC à un site d'environ 150 à 200 bases en amont du promoteur peut conférer ce caractère thermoinductible. Le motif GCACTC 9N GAGTGC peut aussi, dans certains cas, être inséré à un site qui est, au moins en partie, au sein du promoteur. Ceci étant, le site d'insertion doit être choisi de telle manière que la simple insertion du motif ne perturbe pas l'activité du promoteur au-delà de son effet thermoinductible. Il est important de ne pas modifier les séquences -10 et -35 du promoteur lors de cette insertion. Le caractère thermoinductible de la séquence régulatrice d'initiation de transcription ainsi produite peut être vérifié en appliquant la méthode décrite ci-dessus.

Lors de la recherche des promoteurs, les inventeurs ont étudié la réponse au choc thermique de différentes espèces de Streptomyces. Outre les principales protéines de choc thermique de poids moléculaire de 90-100, 70 et 56-58 kDa, une protéine de 16 à 18 kDA a été observée chez chacune des espèces testées. Cette protéine (appelée HSP18) est produite à un taux très élevé chez Streptomyces albus lors du passage de la culture de 30 à 37°C et peut constituer jusqu'à 10 % des protéines totales. L'induction par choc thermique des protéines de 70 et 90-100 kDa est transitoire, tandis que celle des protéines de 56-58 kDa et 18 kDa est constitutive, la production étant maintenue à des températures élevées.

La protéine nommée HSP18 a été purifiée et caractérisée. Ses propriétés ne sont pas semblables à celles d'autres protéines de choc thermique. Par exemple, outre sa taille relativement petite, et sa régulation constitutive a haute température, elle possède un point isoélectrique de plus de 9. Ce point isoélectrique très élevé n'est pourtant pas reflété par sa composition en acides aminés (voir tableau 2).

De plus, la détermination de sa composition en acides aminés a révélé une teneur en méthionine plutôt basse, qui n'est pas cohérente avec les résultats des expériences d'incorporation de [$^{35}$S] méthionine (voir tableau 1). Ces observations suggèrent que la protéine HSP18 subit une modification qui a lieu après la traduction.

La protéine HSP18 ne réagit pas avec des anticorps polyclonaux contre la protéine GroEL d'E. coli, ni avec des anticorps monoclonaux spécifiques pour la protéine de choc thermique de 65 kDa de Mycobacterium leprae.

5

Une étude de transcription du gène "groEL-1" codant pour la protéine HSP18 a révélé que HSP 18 est, en fait, une protéine tronquée. Le gène groEL-1 code en réalité pour une protéine de 56 kDa qui est modifiée après traduction et donne ainsi naissance à la protéine de 18 kDa.

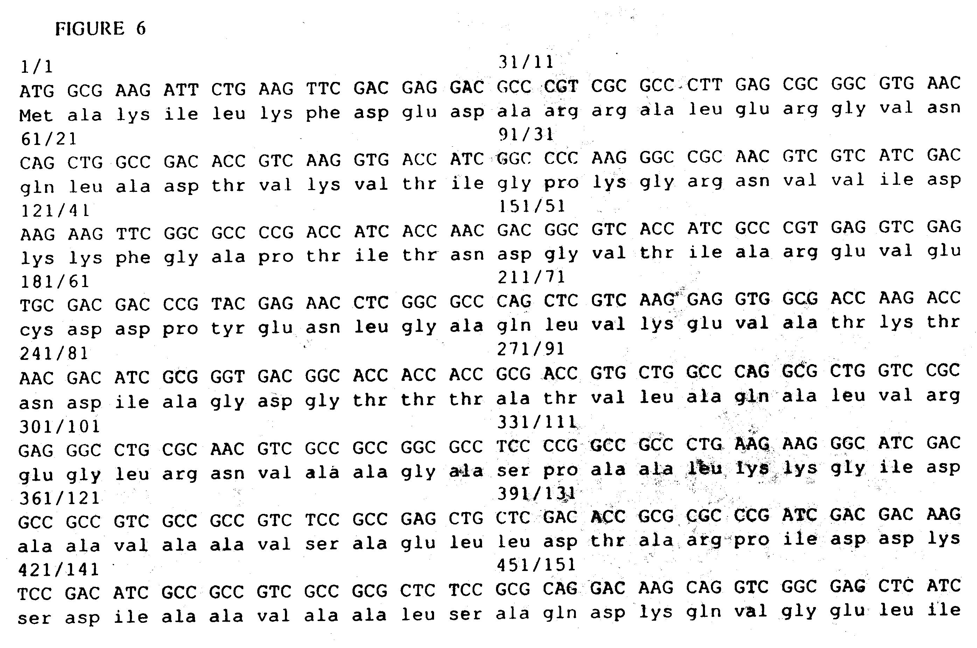

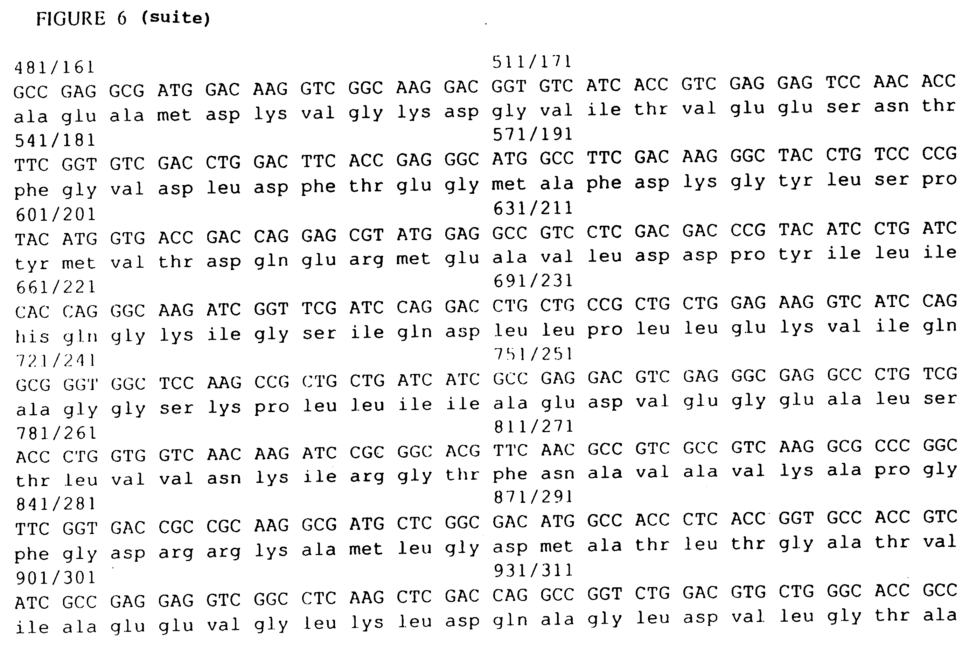

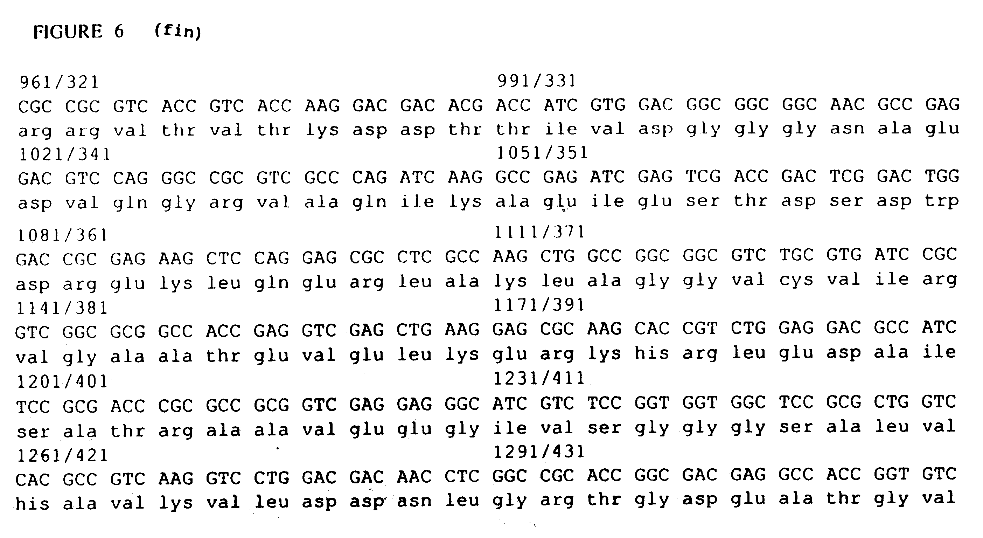

La figure 6 montre la séquence partielle du gène groEL-1 et sa traduction en acides aminés. Cette séquence corrobore les séquences déterminées par dégradation d'Edman sur la HSP18. Il manque à la séquence de la figure 6 l'extrémité COOH terminale de la protéine de 56 kDa. La séquence de la protéine de 18 kDa est comprise dans cette séquence-mère, leurs deux extrémités NH$_2$ terminales coïncidant (acide aminé n° 1). HSP18 s'étend au maximum jusqu'à l'acide aminé 170 environ.

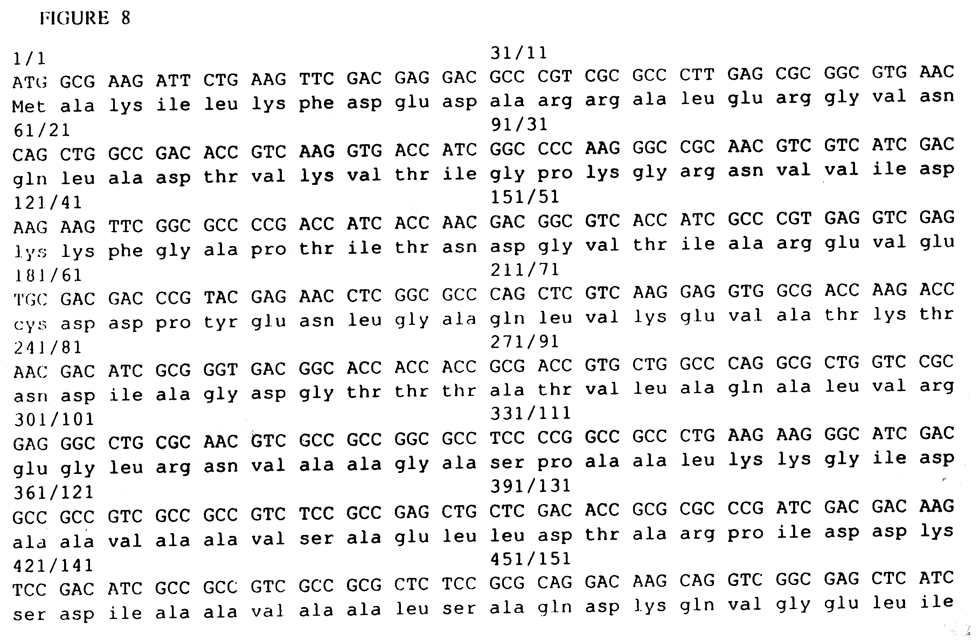

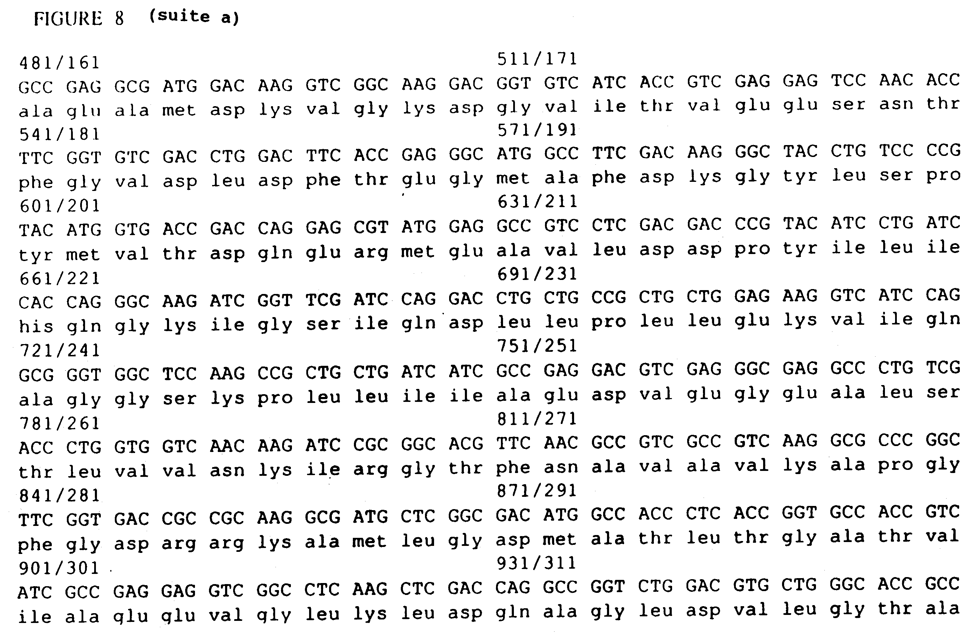

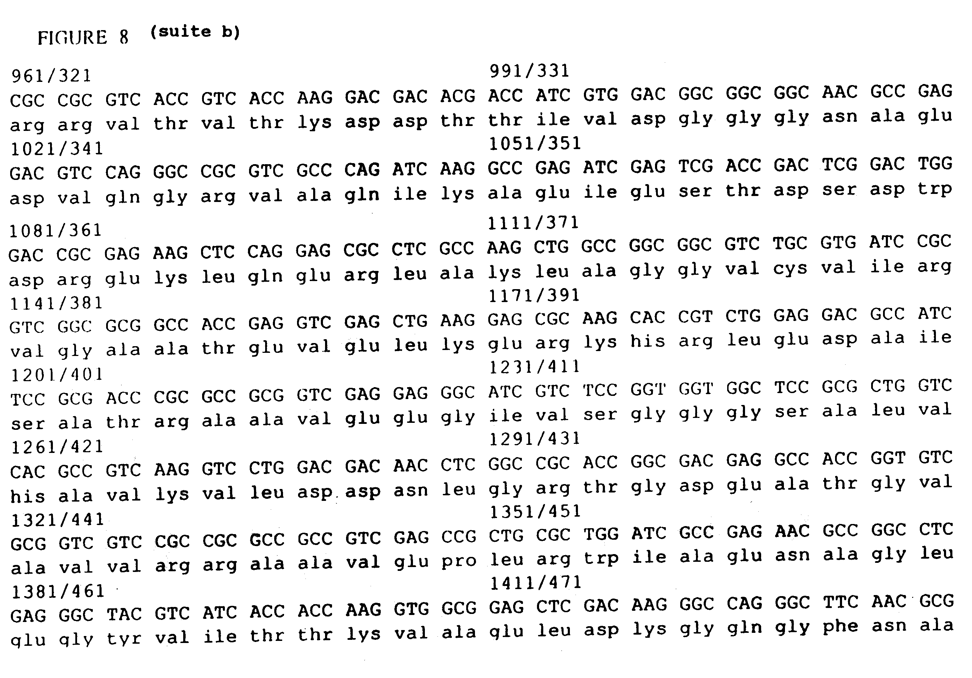

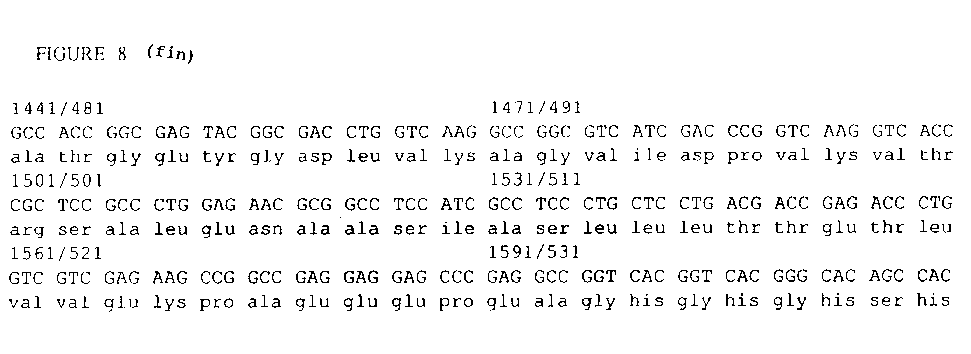

La figure 8 donne la séquence complète de la protéine codée par le gène groEL1, qui comprend, comme la figure présentée à la figure 6, la protéine HSP18.

L'invention vise un polypeptide de choc thermique comportant soit (i) la séquence d'acides aminés indiquée dans la figure 6 ou la séquence répondant à l'enchaînement d'acides aminés de la figure 8, soit (ii) une séquence présentant au moins 85 % d'homologie avec cette séquence, soit (iii) une partie de la séquence (i) ou (ii) comprenant l'extrémité NH$_2$ et s'étendant jusqu'aux environs de l'acide aminé 170, le polypeptide (iii) ayant une taille d'environ 18 kDa et un point isoélectrique très basique d'environ 9.

Par analogie avec la fonction d'autres protéines du type GroEL, il est probable que la HSP18 soit essentielle pour la survie de la cellule et joue un rôle de "chaperon moléculaire", c'est-à-dire se fixe d'une manière transitoire sur des polypeptides naissants, empêchant l'agrégation de protéines insolubles et permettant le repliement ainsi que le transport à travers la membrane cellulaire. Il est également possible que la HSP18 soit impliquée dans la résistance de la souche à ses propres antibiotiques ou dans la thermotolérance.

L'invention vise aussi, selon un aspect particulier, un polypeptide contenant la région COOH-terminale de la protéine GroEL1 telle que présentée à la figure 8. Un polypeptide particulier répondant à cette définition contient la séquence d'acides aminés suivante, ou correspond à cette séquence: Gly His Gly His Gly His Ser His.

La séquence d'acides aminés décrite ci-dessus répond à un enchaînement d'acides aminés original par comparaison avec les séquences peptidiques COOH-terminales connues chez les protéines de choc thermique. Cette séquence COOH-terminale serait impliquée dans la formation de la protéine tronquée de 18 kDa.

Différents aspects de l'invention sont illustrés dans les figures :
- La figure 1 montre, de façon schématique, le clonage des gènes groEL-1, groES et groEL2. Les sites ayant servi pour la construction des plasmides pPM1005 et pPM997 + Neo sont indiqués entre parenthèses.
- La figure 2 montre le promoteur P2 de groEL2 et le promoteur P1 de groES groEL-1.
- La figure 3 montre le vecteur pPM1005, contenant le gène neo de Tn5 sous le contrôle du fragment SmaI de 440 pb de Streptomyces albus. Ce fragment contient le promoteur P1 et les 160 premières paires de bases du gène groES.
- La figure 4 montre le vecteur pPM997 + Neo contenant le gène neo de Tn5 sous le contrôle du fragment BglII / SstI de 800 pb de Streptomyces albus. Ce fragment contient le promoteur P2 et les 183 premières pb du gène groEL2.
- La figure 5 montre la séquence nucléotidique, et la séquence d'acides aminés déduite, du gène structurel groEs et de la protéine GroES.
- La figure 6 illustre la séquence nucléotidique ainsi que la séquence d'acides aminés déduite de la protéine précurseur de HSP18.

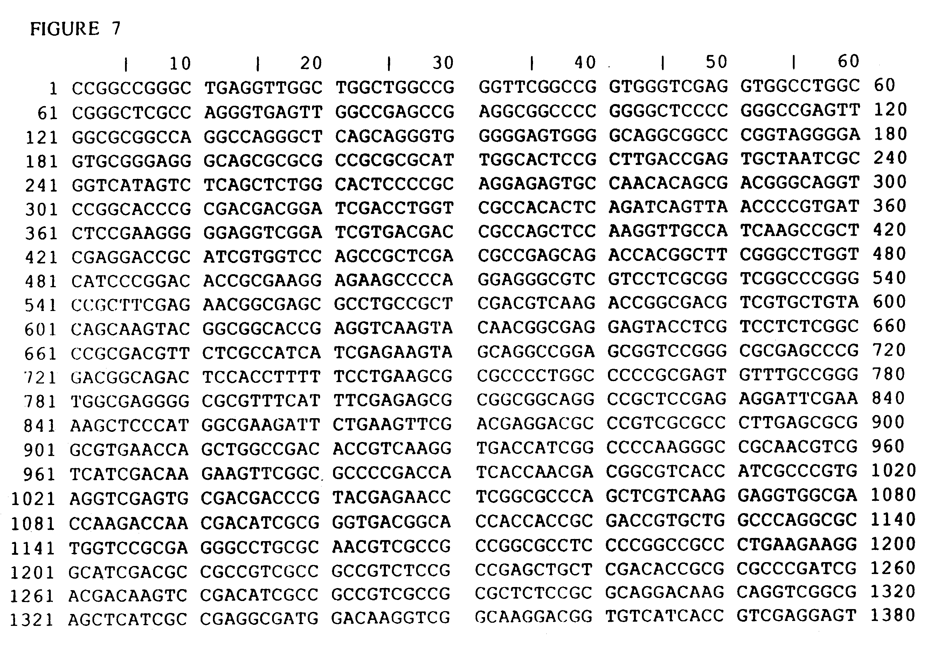

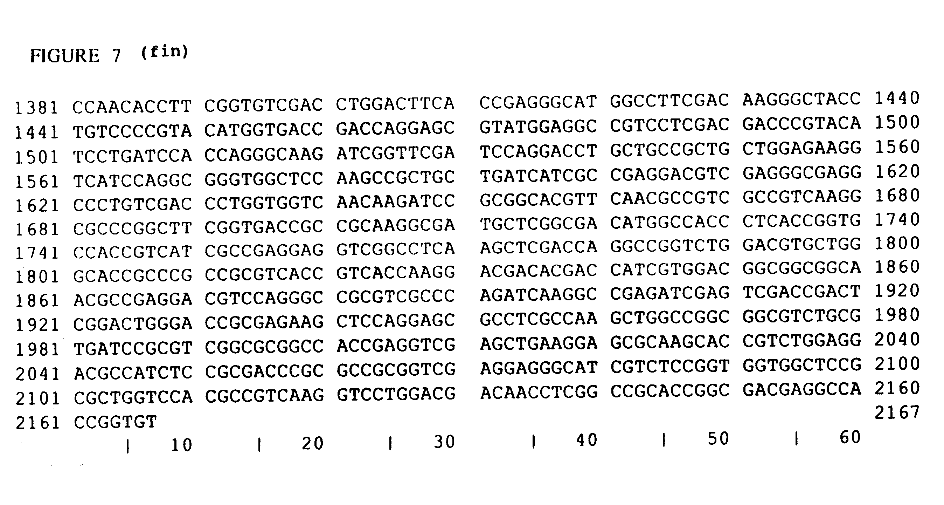

- La figure 7 montre la séquence nucléotidique de l'opéron gro es el avec sa séquence promotrice.
- La figure 8 montre la séquence d'acides aminés complète, codée par le gène groEL1 avec lequel elle est alignée.

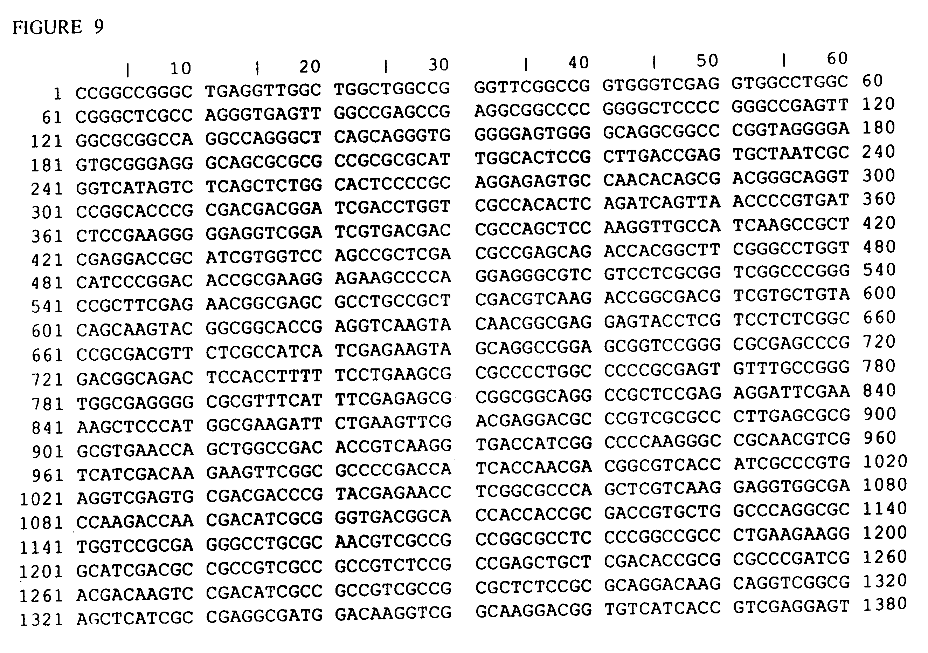

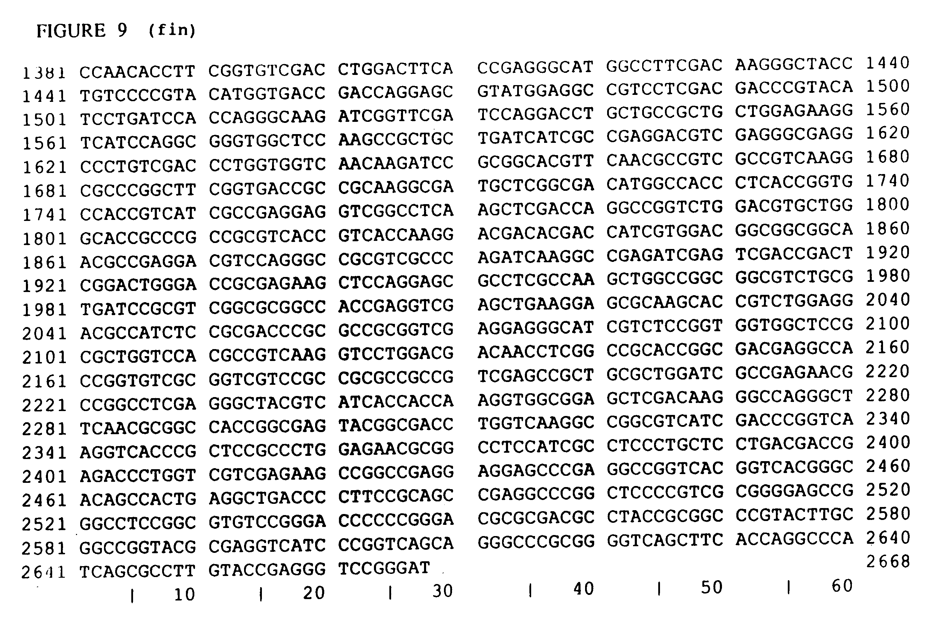

- La figure 9 montre la séquence nucléotidique du gène complet groEL1.

EXEMPLES

**Effet de la température sur la synthèse de protéine chez Streptomyces :**

Les extraits de protéines totales de 15 espèces différentes de Streptomyces ont été préparés avant et après application d'un choc thermique (passage de la température de 30°C à 41°C). Des protéines majeures de choc thermique de 90-100, 70 et 56-58 kDa ont été détectées sur gel de SDS-PAGE, coloré au bleu de Coomassie. De plus, une bande moléculaire correspondant à une protéine de 18 kDa a également été observée chez certaines espèces. Cette protéine était très fortement induite chez Streptomyces albus.

**Propriétés immunologiques des protéines :**

Une analyse "western blot" des protéines sur gel, avec des anticorps monoclonaux contre la protéine de choc thermique de 65 kDa de Mycobacterium leprae et avec des anticorps polyclonaux contre la protéine GroEL d'E.coli, a été effectuée. Aucune des protéines n'a réagi avec les anticorps monoclonaux et seule, les HSPs 56-58 ont réagi avec les anticorps polyclonaux.

**Etude des protéines de choc thermique chez Streptomyces albus :**

La réponse au choc thermique chez Streptomyces albus a été analysée par électrophorèse des protéines totales à 30°C et 41°C. Les protéines ont été marquées pendant 40 minutes. Les acides aminés utilisés étaient [$^{35}$S] méthionine et [$^{14}$C] alanine. Quatre protéines principales de choc thermique ont pu être visualisées. HSP90, qui n'était pas détectable à 30°C, représentait environ 3 % des protéines totales après choc thermique. Le taux de HSP70 a au moins doublé. HSP56-58 présentait une augmentation de 30 % et HPS 18, qui n'était pas décelable avant le choc thermique, représentait 4 à 7 % des protéines totales après le choc (voir tableau 1).

**TABLEAU 1 : QUANTIFICATION DES TAUX DE SYNTHESE DES PROTEINES MAJEURES DE CHOC THERMIQUE APRES MARQUAGE AU $^{14}$C ALANINE ET $^{35}$S METHIONINE**

**Taux de synthèse[a] à 30°C et 41°C**

| Protéine MW[b] | $^{35}$S/30°C | $^{35}$S/41°C | $^{14}$C/30°C | $^{14}$C/41°C |
|---|---|---|---|---|
| 90 | c | 3 | - | 2,9 |
| 70 | 2,7 | 6,4 | 2,3 | 5,8 |
| 56-58 | 6,8 | 8,4 | 6,2 | 9,0 |
| 18 | >0,7? | 6,9 | >0,7? | 3,8 |

a. exprimé sous forme de pourcentage de la densité optique totale (O.D.) mesurée par autoradiographie.
b. poids moléculaire apparent en kDa.
c. pas détecté.

**Etude de HSP18 de Streptomyces albus :**

La protéine de 18 kDa de Streptomyces albus (HSP18), qui est extrêmement basique, a été purifiée et sa composition partielle en acides aminés déterminée (voir tableau 2) :

TABLEAU 2

| COMPOSITION EN ACIDES AMINES DE HSP18 | |
|---|---|
| Asx | 12,2 |
| Thr | 9,4 |
| Ser | 3,2 |
| Glx | 10,8 |
| Ala | 12,1 |
| Cys | 0,0 |
| Met | 0,0 |
| Val | 9,2 |
| Ile | 5,7 |
| Leu | 6,8 |
| Tyr | 1,3 |
| Phe | 1,9 |
| His | 0,3 |
| Lys | 6,9 |
| Arg | 4,5 |
| Gly | 11,0 |
| Pro | 4,4 |

La séquence de l'extrémité $NH_2$ et de deux fragments internes à la protéine ont été déterminés par dégradation d'Edman.

**Synthèse d'oligonucléotides :**

Deux sondes nucléotidiques dégénérées de 30 bases ont été synthétisées en se basant sur la séquence peptidique d'un des fragments internes décrits ci-dessus. La séquence de ce fragment est :
... D - D - P - Y - E - N - L - G - A - Q...
Les sondes deoxyoligonucléotides synthétisées étaient les suivantes :

```
5'    GAC-GAC-CCC-TAC-GAG-AAC-CTG-GGC-GCC-CA        3'OL1
              G              C         T

3'    CTG-CTG-GGG-ATG-CTC-TTG-GAC-CCG-CGG-GTC        5'OL2
              C              G         A
```

**Clonage du gène de la protéine thermoinductible HSP18 :**

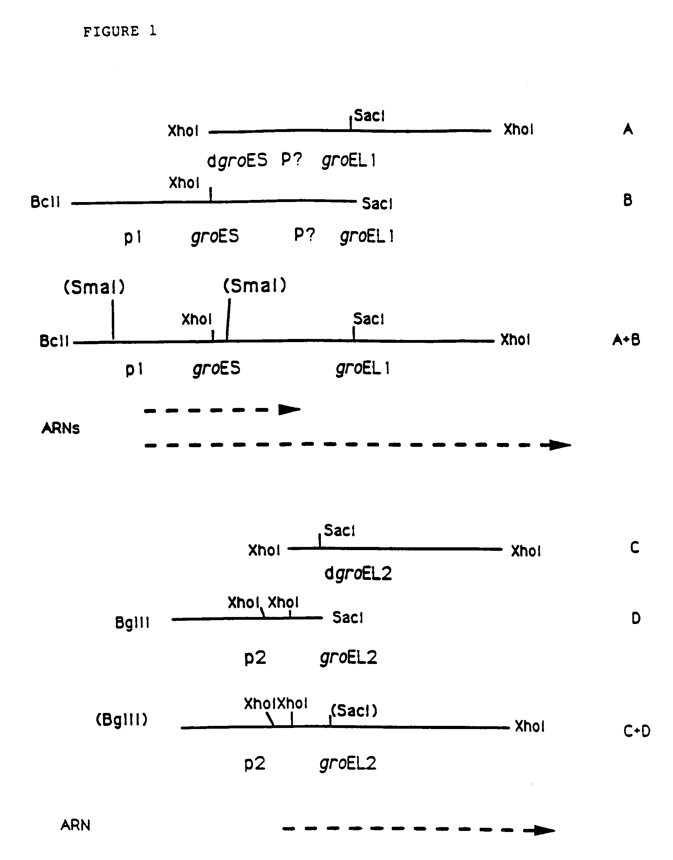

Ces sondes oligonucléotidiques ont permis, après hybridation à 60°C en SSC 5x, de caractériser et de cloner un fragment de restriction Xhol de 1.9kb de Streptomyces albus (voir clonage A de la figure 1).

Ce fragment a été séquencé ; il contient une phase de lecture ouverte qui s'étend à partir d'un ATG en position 430 jusqu'au-delà de la région clonée codant donc pour une protéine de plus de 50 kDa, mais dont l'extrémité NH2 terminale correspond à la séquence nucléotidique déduite de la séquence peptidique d'HSP18. Ce gène a, en outre, sur toute sa longueur une forte homologie de séquence en acides aminés avec la protéine heat-shock groEL d'Escherichia coli et la protéine de 65 kDa de Mycobacterium leprae (75 % d'homologie). Dans un premier temps, ce gène a été appelé "groEL1".

**Démonstration et clonage d'un deuxième gène "groEL-like" chez Streptomyces albus :**

Des hybridations du génome de Streptomyces albus, en utilisant comme sonde la partie 5' du gène de HSP65 de Mycobacterium leprae, ont été effectuées ; cette sonde donne deux signaux après hybridation avec le génome de Streptomyces albus, un fort et un faible et cela quel que soit l'enzyme utilisé pour la digestion de l'ADN. Le signal faible correspond aux signaux obtenus avec les oligonucléotides déduits de HSP18, c'est-à-dire au gène groEL1. Le signal fort correspond à un gène codant pour une autre protéine "groEL-like" de taille canonique (65 kDa). Il y a donc deux gènes groEL-like chez Streptomyces albus.

### Clonage du gène de la protéine heat shock HSP65 de Streptomyces albus :

Le fragment de restriction XhoI de 1,2 kb fortement allumé par la sonde HSP65 de Mycobacterium leprae a été cloné (clonage C figure 1).

La séquence nucléotidique de ce fragment a été déterminée. Le fragment XhoI de 1,2 kb code pour un fragment interne à une protéine homologue à 90 % avec la protéine de 65 kDa de Mycobacterium leprae, en outre les deux gènes "groEl-like" 1 et 2 chez Streptomyces albus sont homologues à 80 %.

Le gène codant pour cette protéine de 65 kDa a été appelé groEL2.

### Etude de la transcription des gènes "groEL-like" et recherche des promoteurs :

Les ARN totaux de la souche Streptomyces albus ont été extraits au cours du temps lors d'une expérience de choc thermique et traités selon la technique de "Northern", puis hybridés avec divers oligonucléotides, synthétisés soit à partir de la séquence de groEL1, soit à partir de la séquence de groEL2 et spécifiques de chacune des régions choisies dans ces deux séquences. Les mêmes filtres de nitrocellulose ont été utilisés dans de nouvelles hybridations avec la totalité des deux fragments clonés. Trois transcrits très fortement thermoinductibles sont observés ; leur taille est respectivement d'environ 2500, 2100 et 650 bases. Celui de 2100 b correspond au transcrit de groEL2 ; celui de 650 b au transcrit d'un gène situé en amont de groEL1 ; celui de 2500 b à la co-transcription de groEL1 et du gène situé en amont de groEL1. Ces résultats indiquaient, d'une part, que les deux gènes groEL1 et groEL2 avaient bien des promoteurs forts et inductibles, et en particulier thermo-inductibles et que, d'autre part, le promoteur de groEL1 n'avait pas été cloné dans le fragment de 1,9 kb. En particulier, ces résultats montrent qu'aucun ARN ne commençait à la boucle notée P? dans la figure 1, séquence postulée comme pouvant servir de promoteur chez Mycobacterium.

En effet, cette boucle renferme deux séquences TTTGCCGGG et TTTCAT qui, en l'absence de travaux de cartographie de promoteur, ont été considérées comme étant respectivement les régions -35 et -10 du promoteur de la protéine de 65 kDa chez Mycobacterium (voir par exemple, J. Gen Microbiol. (1989), 135, 931-939). Ces résultats indiquent que ces deux séquences ne font pas partie du promoteur du gène groEL1 chez Streptomyces albus.

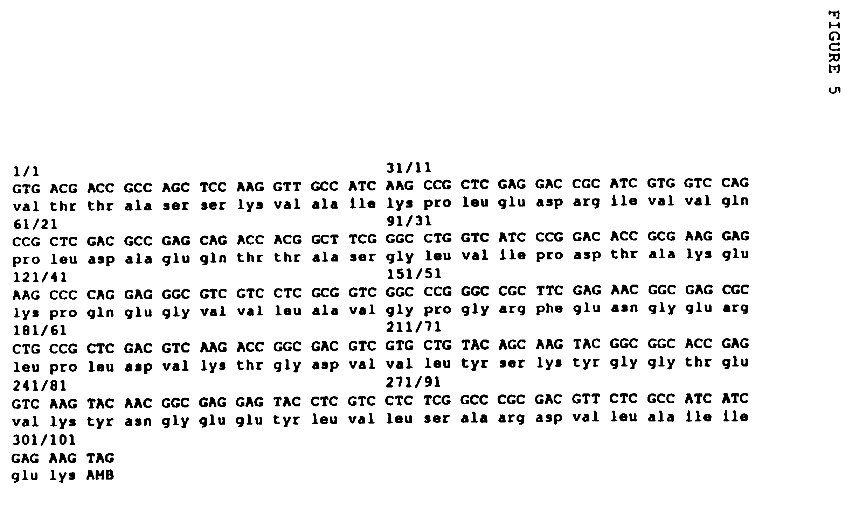

Le promoteur recherché devait se situer en amont d'un gène formant un opéron avec groEL1. Le gène situé en amont de groEL1 a été identifié ; il s'agit d'un gène fortement homologue au gène groES d'E. coli où il forme également un opéron avec groEL1 (voir figure 5).

### Clonage des régions promoteurs des deur gènes groEL1 et groEL :

Deux nouveaux fragments d'ADN de Streptomyces albus, hydrolysés par BclI / SacI (1700 bp) et BglIII / SacI / (900 bp) ont été clonés à l'aide d'oligonucléotides synthétisés à partir de la séquence des fragments précédemment clonés et décrits ci-dessus. Ils recouvrent donc partiellement, respectivement le fragment portant groEL1 et celui portant groEL2 et s'étendent en amont sur environ 800 bp pour chacun d'eux (clonages B et D figure 1).

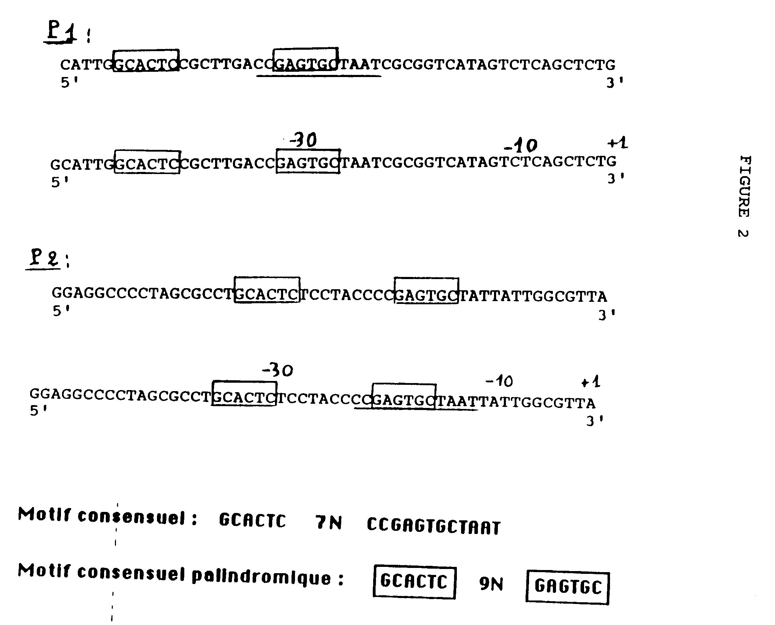

Ces fragments ont été séquencés, puis la caractérisation des promoteurs a été faite par cartographie à la nucléase S1 et par extension d'amorce à la reverse transcriptase. Les promoteurs des deux gènes groEL1 et groEL2 ainsi caractérisés n'ont pas une séquence identique (figure 2), mais ils ont une homologie de structure importante, ils présentent en particulier tous deux le motif palindromique suivant :

GCACTC 9N GAGTGC

Les séquences des deux promoteurs sont les suivantes :

P1 correspondant à l'une des séquences :

CATTGGCACTCCGCTTGACCGAGTGCTAATCGCGGTCATAGTCTCAGCTCTG

5'          3'

ou

GCATTGGCACTCCGCTTGACCGAGTGCTAATCGCGGTCATAGTCTCAGCTCTG

5'          3'

P2 correspondant à l'une des séquences :

GGAGGCCCCTAGCGCCTGCACTCTCCTACCCCGAGTGCTATTATTGGCGTTA

5'          3'

ou

GGAGGCCCCTAGCGCCTGCACTCTCCTACCCCGAGTGCTAATTATTGGCGTTA

5'          3'

**Utilisation des promoteurs de groEL1 et groEL2 pour l'expression d'un gène hétérologue :**

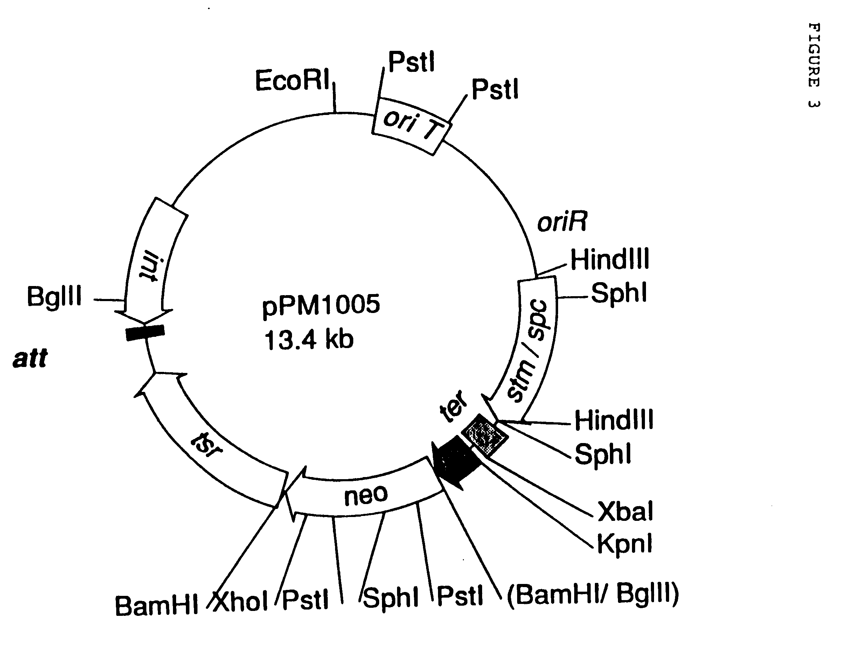

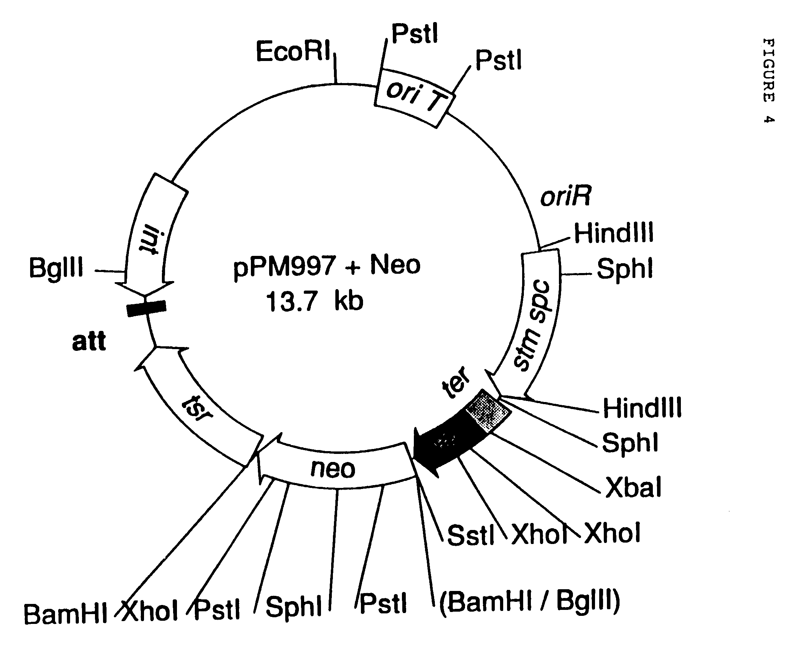

Ces deux promoteurs ont été utilisés pour l'expression du gène hétérologue neo du transposon Tn5 de Klebsiella. Ce gène code pour une aminoside phosphotransferase (APH) conférant la résistance à la néomycine / kanamycine. Ce gène a été cloné en aval des deux promoteurs (figures 3 et 4) puis introduit chez Streptomyces albus et également chez S. lividans. Le gène neo est alors fortement exprimé comme en témoigne l'important niveau de résistance à ces antibiotiques qu'il confère ; en outre, nous avons pu visualiser la synthèse de l'APH dans les extraits bruts après électophorèse sur gel de polyacrylamide et immuno-blot avec des anti-corps anti-APH. Il faut souligner que ces résultats ont été obtenus chez Streptomyces avec un vecteur intégratif. Il n'y a donc dans ces expériences qu'une copie du gène neo et du promoteur étudié par génome. En effet, afin de juger la force du promoteur, il était important de ne pas augmenter artificiellement l'expression de neo en augmentant son nombre de copies, par l'utilisation d'un vecteur à grand nombre de copies.

Les fragments HindIII-BamHI du pPM1005 et SmaI-SmaI du pPM997 ont également été insérés chez Mycobacterium. Le fragment SmaI-SmaI du pPM997 renferme le gène neo, le promoteur P2 et le terminateur.

**Revendications**

1. Séquence nucléotidique recombinante caractérisée en ce qu'elle comprend :
   - une séquence régulatrice de l'initiation de transcription, cette séquence régulatrice comportant un promoteur en association avec le motif GCACTC 9H GAGTGC, dans lequel "N" signifie l'une quelconque des 4 bases thymine, guanine, adénine, et cytosine ;
   - une séquence codant pour un polypeptide, dit "polypeptide hétérologue" autre que celle naturellement associée avec ledit promoteur ;

   ladite séquence codante étant positionnée en aval de ladite séquence régulatrice de l'initiation de transcription, à un site qui, dans des conditions appropriées, permettrait l'expression du polypeptide sous le contrôle dudit promoteur.

2. Séquence nucléotidique selon la revendication 1 caractérisée en ce que le promoteur est un promoteur normalement associé avec une protéine de choc thermique par exemple du type présent chez Actinomycètes.

3. Séquence nucléotidique selon l'une quelconque des revendications 1 ou 2 caractérisée en ce que le motif GCACTC 9N GAGTGC est compris, au moins en partie, au sein de la séquence du promoteur.

**4.** Séquence nucléotidique selon les revendications 2 et 3 caractérisée en ce que le promoteur est sélectionné parmi les séquences suivantes:

P1 correspondant à l'une des séquences :

**CATTGGCACTCCGCTTGACCGAGTGCTAATCGCGGTCATAGTCTCAGCTCTG**

**5'                                                                            3'**

**ou**

**GCATTGGCACTCCGCTTGACCGAGTGCTAATCGCGGTCATAGTCTCAGCTCTG**

**5'                                                                            3'**

P2 correspondant à l'une des séquences :

**GGAGGCCCCTAGCGCCTGCACTCTCCTACCCCGAGTGCTATTATTGGCGTTA**

**5'                                                                            3'**

**ou**

**GGAGGCCCCTAGCGCCTGCACTCTCCTACCCCGAGTGCTAATTATTGGCGTTA**

**5'                                                                            3'**

**5.** Séquence nucléotidique selon la revendication 1 ou 2 caractérisée en ce que le motif GCACTC 9N GAGTGC est distinct de la séquence du promoteur.

**6.** Séquence nucléotidique selon la revendication 5 caractérisée en ce que le motif GCACTC 9N GAGTGC se trouve en amont de la séquence du promoteur, par exemple, environ 150 à 200 bases en amont.

**7.** Vecteur d'expression contenant une séquence nucléotidique recombinante selon l'une quelconque des revendications 1 à 6, et apte à permettre l'expression du polypeptide.

**8.** Vecteur d'expression selon la revendication 7 qui est un plasmide.

**9.** Cellule transformée par le vecteur d'expression selon l'une quelconque des revendications 7 et 8, capable de reconnaître ledit promoteur et d'exprimer ledit polypeptide.

**10.** Cellule selon la revendication 9 qui est une cellule prokaryote par exemple, de l'ordre des Actinomycètes.

**11.** Procédé pour la production d'un polypeptide caractérisé en ce qu'il comprend les étapes suivantes :
- transformation d'une cellule par un vecteur d'expression selon l'une quelconque des revendications 7 ou 8 dans des conditions permettant l'expression dudit polypeptide, ladite cellule étant capable de reconnaître ledit promoteur ;
- récupération du polypeptide exprimé.

**12.** Procédé selon la revendication 11 caractérisé en ce que les conditions permettant l'expression dudit polypeptide comprennent l'application d'un choc thermique.

**13.** Procédé selon l'une quelconque des revendications 11 ou 12 caractérisé en ce que la cellule est une cellule prokaryote par exemple de l'ordre des Actinomycètes, et plus particulièrement du genre Streptomyces.

**14.** Procédé pour conférer un caractère thermoinductible à un promoteur, caractérisé par la juxtaposition d'une part, d'une séquence comprenant le motif GCACTC 9N GAGTGC et, d'autre part, du promoteur; la séquence comprenant le motif GCACTC 9N GAGTGC étant positionnée en amont du promoteur, ou par l'insertion de la séquence comprenant le motif GCACTC 9N GAGTGC à un site qui est, au moins en partie, au sein du promoteur, ce site étant choisi de manière à ce que la simple insertion dudit motif ne perturbe pas l'activité du promoteur.

**15.** Procédé selon la revendication 14 caractérisé en ce que le promoteur est un promoteur présent chez les Actinomycètes, par exemple chez <u>Streptomyces</u> ou <u>Mycobacterium</u>.

**16.** Séquence régulatrice d'initiation de transcription caractérisée en ce qu'elle comporte un promoteur en association avec le motif GCACTC 9N GAGTGC, cette séquence étant exempte de la séquence codante normalement associée à ce promoteur.

**17.** Séquence régulatrice d'initiation de transcription, caractérisée en ce qu'elle comporte une des séquences suivantes :

<u>P1</u> correspondant à l'une des séquences :

CATTGGCACTCCGCTTGACCGAGTGCTAATCGCGGTCATAGTCTCAGCTCTG
5'                                                3'

ou

GCATTGGCACTCCGCTTGACCGAGTGCTAATCGCGGTCATAGTCTCAGCTCTG
5'                                                3'

<u>P2</u> correspondant à l'une des séquences :

GGAGGCCCCTAGCGCCTGCACTCTCCTACCCCGAGTGCTATTATTGGCGTTA
5'                                                3'

ou

GGAGGCCCCTAGCGCCTGCACTCTCCTACCCCGAGTGCTAATTATTGGCGTTA
5'                                                3'

**18.** Polypeptide de choc thermique comportant :
- soit (i) l'une des séquences d'acides aminés indiquées ci-dessous ;
- soit (ii) une séquence d'acides aminés présentant au moins 90 % d'homologie avec cette séquence ;
- soit (iii) une partie de la séquence (i) ou (ii), ladite partie comprenant l'extrémité $NH_2$ terminale et ledit polypeptide ayant une taille d'environ 18kDa et un point isoélectrique de 9 environ ;
- soit (iv) une partie de la séquence (i), ladite partie contenant ou correspondant à la région COOH-terminale : Gly His Gly His Gly His Ser His.

```
1/1                                          31/11
ATG GCG AAG ATT CTG AAG TTC GAC GAG GAC GCC CGT CGC GCC CTT GAG CGC GGC GTG AAC
Met ala lys ile leu lys phe asp glu asp ala arg arg ala leu glu arg gly val asn
61/21                                        91/31
CAG CTG GCC GAC ACC GTC AAG GTG ACC ATC GGC CCC AAG GGC CGC AAC GTC GTC ATC GAC
gln leu ala asp thr val lys val thr ile gly pro lys gly arg asn val val ile asp
121/41                                       151/51
AAG AAG TTC GGC GCC CCG ACC ATC ACC AAC GAC GGC GTC ACC ATC GCC CGT GAG GTC GAG
lys lys phe gly ala pro thr ile thr asn asp gly val thr ile ala arg glu val glu
181/61                                       211/71
TGC GAC GAC CCG TAC GAG AAC CTC GGC GCC CAG CTC GTC AAG GAG GTG GCG ACC AAG ACC
cys asp asp pro tyr glu asn leu gly ala gln leu val lys glu val ala thr lys thr
241/81                                       271/91
AAC GAC ATC GCG GGT GAC GGC ACC ACC ACC GCG ACC GTG CTG GCC CAG GCG CTG GTC CGC
asn asp ile ala gly asp gly thr thr thr ala thr val leu ala gln ala leu val arg
301/101                                      331/111
GAG GGC CTG CGC AAC GTC GCC GCC GGC GCC TCC CCG GCC GCC CTG AAG AAG GGC ATC GAC
glu gly leu arg asn val ala ala gly ala ser pro ala ala leu lys lys gly ile asp
361/121                                      391/131
GCC GCC GTC GCC GCC GTC TCC GCC GAG CTG CTC GAC ACC GCG CGC CCG ATC GAC GAC AAG
ala ala val ala ala val ser ala glu leu leu asp thr ala arg pro ile asp asp lys
421/141                                      451/151
TCC GAC ATC GCC GCC GTC GCC GCG CTC TCC GCG CAG GAC AAG CAG GTC GGC GAG CTC ATC
ser asp ile ala ala val ala ala leu ser ala gln asp lys gln val gly glu leu ile
481/161                                      511/171
GCC GAG GCG ATG GAC AAG GTC GGC AAG GAC GGT GTC ATC ACC GTC GAG GAG TCC AAC ACC
ala glu ala met asp lys val gly lys asp gly val ile thr val glu glu ser asn thr
541/181                                      571/191
TTC GGT GTC GAC CTG GAC TTC ACC GAG GGC ATG GCC TTC GAC AAG GGC TAC CTG TCC CCG
phe gly val asp leu asp phe thr glu gly met ala phe asp lys gly tyr leu ser pro
601/201                                      631/211
TAC ATG GTG ACC GAC CAG GAG CGT ATG GAG GCC GTC CTC GAC GAC CCG TAC ATC CTG ATC
tyr met val thr asp gln glu arg met glu ala val leu asp asp pro tyr ile leu ile
661/221                                      691/231
CAC CAG GGC AAG ATC GGT TCG ATC CAG GAC CTG CTG CCG CTG CTG GAG AAG GTC ATC CAG
his gln gly lys ile gly ser ile gln asp leu leu pro leu leu glu lys val ile gln
721/241                                      751/251
GCG GGT GGC TCC AAG CCG CTG CTG ATC ATC GCC GAG GAC GTC GAG GGC GAG GCC CTG TCG
ala gly gly ser lys pro leu leu ile ile ala glu asp val glu gly glu ala leu ser
781/261                                      811/271
ACC CTG GTG GTC AAC AAG ATC CGC GGC ACG TTC AAC GCC GTC GCC GTC AAG GCG CCC GGC
thr leu val val asn lys ile arg gly thr phe asn ala val ala val lys ala pro gly
841/281                                      871/291
TTC GGT GAC CGC CGC AAG GCG ATG CTC GGC GAC ATG GCC ACC CTC ACC GGT GCC ACC GTC
phe gly asp arg arg lys ala met leu gly asp met ala thr leu thr gly ala thr val
901/301                                      931/311
ATC GCC GAG GAG GTC GGC CTC AAG CTC GAC CAG GCC GGT CTG GAC GTG CTG GGC ACC GCC
ile ala glu glu val gly leu lys leu asp gln ala gly leu asp val leu gly thr ala
961/321                                      991/331
CGC CGC GTC ACC GTC ACC AAG GAC GAC ACG ACC ATC GTG GAC GGC GGC GGC AAC GCC GAG
arg arg val thr val thr lys asp asp thr thr ile val asp gly gly gly asn ala glu
1021/341                                     1051/351
GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp
1081/361                                     1111/371
GAC CGC GAG AAG CTC CAG GAG CGC CTC GCC AAG CTG GCC GGC GGC GTC TGC GTG ATC CGC
asp arg glu lys leu gln glu arg leu ala lys leu ala gly gly val cys val ile arg
1141/381                                     1171/391
GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile
1201/401                                     1231/411
TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val
1261/421                                     1291/431
CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val
```

ou la séquence :

```
1/1                                        31/11
ATG GCG AAG ATT CTG AAG TTC GAC GAG GAC GCC CGT CGC GCC CTT GAG CGC GGC GTG AAC
Met ala lys ile leu lys phe asp glu asp ala arg arg ala leu glu arg gly val asn
61/21                                      91/31
CAG CTG GCC GAC ACC GTC AAG GTG ACC ATC GGC CCC AAG GGC CGC AAC GTC GTC ATC GAC
gln leu ala asp thr val lys val thr ile gly pro lys gly arg asn val val ile asp
121/41                                     151/51
AAG AAG TTC GGC GCC CCG ACC ATC ACC AAC GAC GGC GTC ACC ATC GCC CGT GAG GTC GAG
lys lys phe gly ala pro thr ile thr asn asp gly val thr ile ala arg glu val glu
181/61                                     211/71
TGC GAC GAC CCG TAC GAG AAC CTC GGC GCC CAG CTC GTC AAG GAG GTG GCG ACC AAG ACC
cys asp asp pro tyr glu asn leu gly ala gln leu val lys glu val ala thr lys thr
241/81                                     271/91
AAC GAC ATC GCG GGT GAC GGC ACC ACC ACC GCG ACC GTG CTG GCC CAG GCG CTG GTC CGC
asn asp ile ala gly asp gly thr thr thr ala thr val leu ala gln ala leu val arg
301/101                                    331/111
GAG GGC CTG CGC AAC GTC GCC GCC GGC GCC TCC CCG GCC GCC CTG AAG AAG GGC ATC GAC
glu gly leu arg asn val ala ala gly ala ser pro ala ala leu lys lys gly ile asp
361/121                                    391/131
GCC GCC GTC GCC GCC GTC TCC GCC GAG CTG CTC GAC ACC GCG CGC CCG ATC GAC GAC AAG
ala ala val ala ala val ser ala glu leu leu asp thr ala arg pro ile asp asp lys
421/141                                    451/151
TCC GAC ATC GCC GCC GTC GCC GCG CTC TCC GCG CAG GAC AAG CAG GTC GGC GAG CTC ATC
ser asp ile ala ala val ala ala leu ser ala gln asp lys gln val gly glu leu ile
481/161                                    511/171
GCC GAG GCG ATG GAC AAG GTC GGC AAG GAC GGT GTC ATC ACC GTC GAG GAG TCC AAC ACC
ala glu ala met asp lys val gly lys asp gly val ile thr val glu glu ser asn thr
541/181                                    571/191
TTC GGT GTC GAC CTG GAC TTC ACC GAG GGC ATG GCC TTC GAC AAG GGC TAC CTG TCC CCG
phe gly val asp leu asp phe thr glu gly met ala phe asp lys gly tyr leu ser pro
601/201                                    631/211
TAC ATG GTG ACC GAC CAG GAG CGT ATG GAG GCC GTC CTC GAC GAC CCG TAC ATC CTG ATC
tyr met val thr asp gln glu arg met glu ala val leu asp asp pro tyr ile leu ile
661/221                                    691/231
CAC CAG GGC AAG ATC GGT TCG ATC CAG GAC CTG CTG CCG CTG CTG GAG AAG GTC ATC CAG
his gln gly lys ile gly ser ile gln asp leu leu pro leu leu glu lys val ile gln
721/241                                    751/251
GCG GGT GGC TCC AAG CCG CTG CTG ATC ATC GCC GAG GAC GTC GAG GGC GAG GCC CTG TCG
ala gly gly ser lys pro leu leu ile ile ala glu asp val glu gly glu ala leu ser
781/261                                    811/271
ACC CTG GTG GTC AAC AAG ATC CGC GGC ACG TTC AAC GCC GTC GCC GTC AAG GCG CCC GGC
thr leu val val asn lys ile arg gly thr phe asn ala val ala val lys ala pro gly
841/281                                    871/291
TTC GGT GAC CGC CGC AAG GCG ATG CTC GGC GAC ATG GCC ACC CTC ACC GGT GCC ACC GTC
phe gly asp arg arg lys ala met leu gly asp met ala thr leu thr gly ala thr val
901/301                                    931/311
ATC GCC GAG GAG GTC GGC CTC AAG CTC GAC CAG GCC GGT CTG GAC GTG CTG GGC ACC GCC
ile ala glu glu val gly leu lys leu asp gln ala gly leu asp val leu gly thr ala
961/321                                    991/331
CGC CGC GTC ACC GTC ACC AAG GAC GAC ACG ACC ATC GTG GAC GGC GGC GGC AAC GCC GAG
arg arg val thr val thr lys asp asp thr thr ile val asp gly gly gly asn ala glu
```

1021/341              1051/351

GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp

1081/361              1111/371

GAC CGC GAG AAG CTC CAG GAG CGC CTC GCC AAG CTG GCC GGC GGC GTC TGC GTG ATC CGC
asp arg glu lys leu gln glu arg leu ala lys leu ala gly gly val cys val ile arg

1141/381              1171/391

GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile

1201/401              1231/411

TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val

1261/421              1291/431

CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val

1321/441              1351/451

GCG GTC GTC CGC CGC GCC GCC GTC GAG CCG CTG CGC TGG ATC GCC GAG AAC GCC GGC CTC
ala val val arg arg ala ala val glu pro leu arg trp ile ala glu asn ala gly leu

1381/461              1411/471

GAG GGC TAC GTC ATC ACC ACC AAG GTG GCG GAG CTC GAC AAG GGC CAG GGC TTC AAC GCG
glu gly tyr val ile thr thr lys val ala glu leu asp lys gly gln gly phe asn ala

1441/481              1471/491

GCC ACC GGC GAG TAC GGC GAC CTG GTC AAG GCC GGC GTC ATC GAC CCG GTC AAG GTC ACC
ala thr gly glu tyr gly asp leu val lys ala gly val ile asp pro val lys val thr

1501/501              1531/511

CGC TCC GCC CTG GAG AAC GCG GCC TCC ATC GCC TCC CTG CTC CTG ACG ACC GAG ACC CTG
arg ser ala leu glu asn ala ala ser ile ala ser leu leu leu thr thr glu thr leu

1561/521              1591/531

GTC GTC GAG AAG CCG GCC GAG GAG GAG CCC GAG GCC GGT CAC GGT CAC GGG CAC AGC CAC
val val glu lys pro ala glu glu glu pro glu ala gly his gly his gly his ser his

**19.** Polypeptide selon la revendication 18 caractérisé par une séquence d'acides aminés qui s'étend de l'acide aminé numéroté 1 à l'acide aminé 170 au maximum.

**20.** Séquence d'acide nucléique codant pour une protéine de choc thermique, selon l'une quelconque des revendications 18 ou 19.

**21.** Séquence d'acide nucléique selon la revendication 20, caractérisée en ce qu'elle comprend au moins un fragment de la séquence indiquée dans la revendication 18.

**Claims**

**1.** Recombinant nucleotide sequence, characterized in that it comprises:
- a regulatory sequence for initiating transcription, this regulatory sequence comprising of a promoter in association with the motif GCACTC 9N GAGTGC, in which "N" signifies any of the 4 bases thymine, guanine, adenine and cytosine;
- a sequence encoding a polypeptide, termed "heterologous polypeptide", which is different from that which is naturally associated with the said promoter;
- the said coding sequence being located downstream of the said regulatory sequence for initiating transcription at a site which, under suitable conditions, would permit expression of the polypeptide under the control of the said promoter.

**2.** Nucleotide sequence according to Claim 1, characterized in that the promoter is a promoter which is normally associated with a heat shock protein, for example of the type present in Actinomycetes.

**3.** Nucleotide sequence according to either of Claims 1 or 2, characterized in that the motif GCACTC 9N GAGTGC is included, at least in part, within the sequence of the promoter.

4. Nucleotide sequence according to Claims 2 and 3, characterized in that the promoter is selected from among the following sequences:

P1 corresponding to one of the sequences:

```
CATTGGCACTCCGCTTGACCGAGTGCTAATCGCGGTCATAGTCTCAGCTCTG
5'                                                  3'
```

or

```
GCATTGGCACTCCGCTTGACCGAGTGCTAATCGCGGTCATAGTCTCAGCTCTG
5'                                                   3'
```

P2 corresponding to one of the sequences:

```
GGAGGCCCCTAGCGCCTGCACTCTCCTACCCCGAGTGCTATTATTGGCGTTA
5'                                                  3'
```

or

```
GGAGGCCCCTAGCGCCTGCACTCTCCTACCCCGAGTGCTAATTATTGGCGTTA
5'                                                   3'
```

5. Nucleotide sequence according to Claim 1 or 2, characterized in that the motif GCACTC 9N GAGTGC is distinct from the promoter sequence.

6. Nucleotide sequence according to Claim 5, characterized in that the motif GCACTC 9N GAGTGC is located upstream of the promoter sequence, for example approximately 150 to 200 bases upstream.

7. Expression vector containing a recombinant nucleotide sequence according to any one of Claims 1 to 6, and capable of allowing expression of the polypeptide.

8. Expression vector according to Claim 7, which is a plasmid.

9. Cell which is transformed by the expression vector according to either of Claims 7 or 8, and which is capable of recognizing the said promoter and of expressing the said polypeptide.

10. Cell according to Claim 9, which is a prokaryotic cell, for example from the order of the Actinomycetes.

11. Process for producing a polypeptide, characterized in that it comprises the following steps:
    - transformation of a cell by an expression vector according to either of Claims 7 or 8 under conditions which permit expression of the said polypeptide, the said cell being capable of recognizing the said promoter;
    - recovery of the expressed polypeptide.

12. Process according to Claim 11, characterized in that the conditions which permit expression of the said polypeptide include applying a heat shock.

13. Process according to either of Claims 11 or 12, characterized in that the cell is a prokaryotic cell, for example from the order of the Actinomycetes, and, more specifically, from the genus Streptomyces.

14. Process for imparting a thermoinducible character to a promoter, characterized by the juxtaposition, on the one hand, of a sequence including the motif GCACTC 9N GAGTGC and, on the other hand, of the

16

promoter; the sequence including the motif GCACTC 9N GAGTGC being located upstream of the promoter, or by insertion of the sequence including the motif GCACTC 9N GAGTGC at a site which is, at least in part, within the promoter, this site being selected such that the simple insertion of the said motif does not interfere with the activity of the promoter.

15. Process according to Claim 14, characterized in that the promoter is a promoter which is present in the Actinomycetes, for example in Streptomyces or Mycobacterium.

16. Regulatory sequence for initiating transcription, characterized in that it comprises of a promoter in association with the motif GCACTC 9N GAGTGC, this sequence being free of the coding sequence which is normally associated with this promoter.

17. Regulatory sequence for initiating transcription, characterized in that it consists of one of the following sequences:

P1 corresponding to one of the sequences:

```
CATTGGCACTCCGCTTGACCGAGTGCTAATCGCGGTCATAGTCTCAGCTCTG
5'                                                    3'
or
GCATTGGCACTCCGCTTGACCGAGTGCTAATCGCGGTCATAGTCTCAGCTCTG
5'                                                    3'
```

P2 corresponding to one of the sequences:

```
GGAGGCCCCTAGCGCCTGCACTCTCCTACCCCGAGTGCTATTATTGGCGTTA
5'                                                    3'
or
GGAGGCCCCTAGCGCCTGCACTCTCCTACCCCGAGTGCTAATTATTGGCGTTA
5'                                                    3'
```

18. Heat shock polypeptide consisting of:
   - either (i) one of the amino acid sequences indicated below;
   - or (ii) an amino acid sequence exhibiting at least 90 % homology with this sequence;
   - or (iii) a part of the sequence (i) or (ii), the said part comprising the NH$_2$ terminal end and the said polypeptide having a size of approximately 18kDa and an isoelectric point of approximately 9;
   - or (iv) a part of the sequence (i), the said part containing, or corresponding to, the COOH terminal region: Gly His Gly His Gly His Ser His.

```
1/1                                    31/11
ATG GCG AAG ATT CTG AAG TTC GAC GAG GAC  GCC CGT CGC GCC CTT GAG CGC GGC GTG AAC
Met ala lys ile leu lys phe asp glu asp  ala arg arg ala leu glu arg gly val asn
61/21                                  91/31
CAG CTG GCC GAC ACC GTC AAG GTG ACC ATC  GGC CCC AAG GGC CGC AAC GTC GTC ATC GAC
gln leu ala asp thr val lys val thr ile  gly pro lys gly arg asn val val ile asp
121/41                                 151/51
AAG AAG TTC GGC GCC CCG ACC ATC ACC AAC  GAC GGC GTC ACC ATC GCC CGT GAG GTC GAG
lys lys phe gly ala pro thr ile thr asn  asp gly val thr ile ala arg glu val glu
181/61                                 211/71
TGC GAC GAC CCG TAC GAG AAC CTC GGC GCC  CAG CTC GTC AAG GAG GTG GCG ACC AAG ACC
cys asp asp pro tyr glu asn leu gly ala  gln leu val lys glu val ala thr lys thr
241/81                                 271/91
AAC GAC ATC GCG GGT GAC GGC ACC ACC ACC  GCG ACC GTG CTG GCC CAG GCG CTG GTC CGC
asn asp ile ala gly asp gly thr thr thr  ala thr val leu ala gln ala leu val arg
301/101                                331/111
GAG GGC CTG CGC AAC GTC GCC GCC GGC GCC  TCC CCG GCC GCC CTG AAG AAG GGC ATC GAC
glu gly leu arg asn val ala ala gly ala  ser pro ala ala leu lys lys gly ile asp
361/121                                391/131
GCC GCC GTC GCC GCC GTC TCC GCC GAG CTG  CTC GAC ACC GCG CGC CCG ATC GAC GAC AAG
ala ala val ala ala val ser ala glu leu  leu asp thr ala arg pro ile asp asp lys
421/141                                451/151
TCC GAC ATC GCC GCC GTC GCC GCG CTC TCC  GCG CAG GAC AAG CAG GTC GGC GAG CTC ATC
ser asp ile ala ala val ala ala leu ser  ala gln asp lys gln val gly glu leu ile
481/161                                511/171
GCC GAG GCG ATG GAC AAG GTC GGC AAG GAC  GGT GTC ATC ACC GTC GAG GAG TCC AAC ACC
ala glu ala met asp lys val gly lys asp  gly val ile thr val glu glu ser asn thr
541/181                                571/191
TTC GGT GTC GAC CTG GAC TTC ACC GAG GGC  ATG GCC TTC GAC AAG GGC TAC CTG TCC CCG
phe gly val asp leu asp phe thr glu gly  met ala phe asp lys gly tyr leu ser pro
601/201                                631/211
TAC ATG GTG ACC GAC CAG GAG CGT ATC GAG  GCC GTC CTC GAC GAC CCG TAC ATC CTG ATC
tyr met val thr asp gln glu arg met glu  ala val leu asp asp pro tyr ile leu ile
661/221                                691/231
CAC CAG GGC AAG ATC GGT TCG ATC CAG GAC  CTG CTG CCG CTG CTG GAG AAG GTC ATC CAG
his gln gly lys ile gly ser ile gln asp  leu leu pro leu leu glu lys val ile gln
721/241                                751/251
GCG GGT GGC TCC AAG CCG CTG CTG ATC ATC  GCC GAG GAC GTC GAG GGC GAG GCC CTG TCG
ala gly gly ser lys pro leu leu ile ile  ala glu asp val glu gly glu ala leu ser
781/261                                811/271
ACC CTG GTG GTC AAC AAG ATC CGC GGC ACG  TTC AAC GCC GTC GCC GTC AAG GCG CCC CGC
thr leu val val asn lys ile arg gly thr  phe asn ala val ala val lys ala pro gly
841/281                                871/291
TTC GGT GAC CGC CGC AAG GCG ATG CTC GGC  GAC ATG GCC ACC CTC ACC GGT GCC ACC GTC
phe gly asp arg arg lys ala met leu gly  asp met ala thr leu thr gly ala thr val
901/301                                931/311
ATC GCC GAG GAG GTC GGC CTC AAG CTC GAC  CAG GCC GGT CTG GAC GTG CTG GGC ACC GCC
ile ala glu glu val gly leu lys leu asp  gln ala gly leu asp val leu gly thr ala
```

```
961/321                                           991/331
CGC CGC GTC ACC GTC ACC AAG GAC GAC ACG ACC ATC GTG GAC GGC GGC GGC AAC GCC GAG
arg arg val thr val thr lys asp asp thr thr ile val asp gly gly gly asn ala glu
1021/341                                          1051/351
GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp
1081/361                                          1111/371
GAC CGC GAG AAG CTC CAG GAG CGC CTC GCC AAG CTG GCC GGC GGC GTC TGC GTC ATC CGC
asp arg glu lys leu gln glu arg leu ala lys leu ala gly gly val cys val ile arg
1141/381                                          1171/391
GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile
1201/401                                          1231/411
TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val
1261/421                                          1291/431
CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val
```

or the sequence:

```
1/1                                               31/11
ATG GCC AAC ATT CTG AAG TTC GAC GAG GAC GCC CGT CGC GCC CTT GAG CGC GGC GTC AAC
Met ala lys ile leu lys phe asp glu asp ala arg arg ala leu glu arg gly val asn
61/21                                             91/31
CAG CTG GCC GAC ACC GTC AAG GTG ACC ATC GGC CCC AAG GGC CGC AAC GTC GTC ATC GAC
gln leu ala asp thr val lys val thr ile gly pro lys gly arg asn val val ile asp
121/41                                            151/51
AAG AAG TTC GGC GCC CCG ACC ATC ACC AAC GAC GGC GTC ACC ATC GCC CGT GAG GTC GAG
lys lys phe gly ala pro thr ile thr asn asp gly val thr ile ala arg glu val glu
181/61                                            211/71
TGC GAC GAC CCG TAC GAG AAC CTC GGC GCC CAG CTC GTC AAG GAG GTG GCG ACC AAG ACC
cys asp asp pro tyr glu asn leu gly ala gln leu val lys glu val ala thr lys thr
241/81                                            271/91
AAC GAC ATC GCG GGT GAC GGC ACC ACC ACC GCG ACC GTG CTG GCC CAG GCG CTG GTC CGC
asn asp ile ala gly asp gly thr thr thr ala thr val leu ala gln ala leu val arg
301/101                                           331/111
GAG GGC CTG CGC AAC GTC GCC GCC GGC GCC TCC CCG GCC GCC CTG AAG AAG GGC ATC GAC
glu gly leu arg asn val ala ala gly ala ser pro ala ala leu lys lys gly ile asp
361/121                                           391/131
GCC GCG GTC GCC GCC GTC TCC GCC GAG CTG CTC GAC ACC GCG CGC CCG ATC GAC GAC AAG
ala ala val ala ala val ser ala glu leu leu asp thr ala arg pro ile asp asp lys
421/141                                           451/151
TCC GAC ATC GCC GCC GTC GCC GCG CTC TCC GCG CAG GAC AAG CAG GTC GGC GAG CTC ATC
ser asp ile ala ala val ala ala leu ser ala gln asp lys gln val gly glu leu ile
481/161                                           511/171
GCC GAG GCG ATG GAC AAG GTC GCC AAG GAC GGT CTC ATC ACC GTC GAG GAG TCC AAC ACC
ala glu ala met asp lys val gly lys asp gly val ile thr val glu glu ser asn thr
541/181                                           571/191
TTC GGT GTC GAC CTG GAC TTC ACC GAG GCC ATG GCC TTC GAC AAG GGC TAC CTG TCC CCG
phe gly val asp leu asp phe thr glu gly met ala phe asp lys gly tyr leu ser pro
601/201                                           631/211
TAC ATG GTG ACC GAC CAG GAG CGT ATG GAG GCC GTC CTC GAC GAC CCG TAC ATC CTG ATC
tyr met val thr asp gln glu arg met glu ala val leu asp asp pro tyr ile leu ile
661/221                                           691/231
CAC CAG GGC AAG ATC GGT TCG ATC CAG GAC CTG CTG CCG CTG CTG GAG AAG GTC ATC CAG
his gln gly lys ile gly ser ile gln asp leu leu pro leu leu glu lys val ile gln
721/241                                           751/251
GCG GGT GGC TCC AAG CCG CTG CTG ATC ATC GCC GAG GAC GTC GAG GGC GAG GCC CTG TCG
ala gly gly ser lys pro leu leu ile ile ala glu asp val glu gly glu ala leu ser
781/261                                           811/271
ACC CTG GTG GTC AAC AAG ATC CGC GGC ACG TTC AAC GCC GTC GCC GTC AAG GCG CCC GGC
thr leu val val asn lys ile arg gly thr phe asn ala val ala val lys ala pro gly
```

```
841/281                                  871/291
TTC GGT GAC CGC CGC AAG GCG ATG CTC GGC GAC ATG GCC ACC CTC ACC GGT GCC ACC GTC
phe gly asp arg arg lys ala met leu gly asp met ala thr leu thr gly ala thr val
901/301                                  931/311
ATC GCC GAG GAG GTC GGC CTC AAG CTC GAC CAG GCC GGT CTG GAC GTG CTG GGC ACC GCC
ile ala glu glu val gly leu lys leu asp gln ala gly leu asp val leu gly thr ala
961/321                                  991/331
CGC CGC GTC ACC GTC ACC AAG GAC GAC ACG ACC ATC GTC GAC GGC GGC GGC AAC GCC GAG
arg arg val thr val thr lys asp asp thr thr ile val asp gly gly gly asn ala glu

1021/341                                 1051/351
GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp
1081/361                                 1111/371
GAC CGC GAG AAG CTC CAG GAG CGC CTC GCC AAG CTG GCC GGC GGC CTC TGC GTG ATC CGC
asp arg glu lys leu gln glu arg leu ala lys leu ala gly gly val cys val ile arg
1141/381                                 1171/391
GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile
1201/401                                 1231/411
TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val
1261/421                                 1291/431
CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val
1321/441                                 1351/451
GCG GTC GTC CGC CGC GCC GCC GTC GAG CCG CTG CGC TGG ATC GCC GAG AAC GCC GCC CTC
ala val val arg arg ala ala val glu pro leu arg trp ile ala glu asn ala gly leu
1381/461                                 1411/471
GAG GGC TAC GTC ATC ACC ACC AAG GTG GCG GAG CTC GAC AAG GGC CAG GGC TTC AAC GCG
glu gly tyr val ile thr thr lys val ala glu leu asp lys gly gln gly phe asn ala
1441/481                                 1471/491
CCC ACC GGC GAG TAC GCC GAC CTG GTC AAG GCC GGC GTC ATC GAC CCG GTC AAG GTC ACC
ala thr gly glu tyr gly asp leu val lys ala gly val ile asp pro val lys val thr
1501/501                                 1531/511
CGC TCC GCC CTG GAG AAC GCG GCC TCC ATC GCC TCC CTG CTC CTG ACG ACC GAG ACC CTG
arg ser ala leu glu asn ala ala ser ile ala ser leu leu leu thr thr glu thr leu
1561/521                                 1591/531
GTC GTC GAG AAG CCG GCC GAG GAG CAG CCC GAG GCC GGT CAC GGT CAC GCG CAC AGC CAC
val val glu lys pro ala glu glu glu pro glu ala gly his gly his gly his ser his
```

**19.** Polypeptide according to Claim 18, characterized by an amino acid sequence which extends from the amino acid numbered 1 to, at most, the amino acid 170.

**20.** Nucleic acid sequence encoding a heat shock protein, according to either of Claims 18 or 19.

**21.** Nucleic acid sequence according to Claim 20, characterized in that it comprises at least a fragment of the sequence indicated in Claim 18.

## Patentansprüche

**1.** Rekombinante Nukleotidsequenz,
dadurch gekennzeichnet, daß sie enthält:
- eine Regulationssequenz der Initiation der Transkription, wobei diese Regulationssequenz einen Promotor in Verbindung mit dem Motiv GCACTC 9N GAGTGC enthält, in dem "N" eine beliebige der 4 Basen Thymin, Guanin, Adenin und Cytosin bezeichnet,
- eine Sequenz, die für ein Polypeptid, genannt "heterologes Polypeptid", das sich von dem natürlich mit diesem Promotor assoziierten Polypeptid unterscheidet, kodiert,

wobei die kodierende Sequenz stromabwärts von der Regulationssequenz der Initiation der Transkription an einer Stelle angeordnet ist, die unter geeigneten Bedingungen die Expression des Polypeptids unter der Kontrolle des Promotors erlaubt.

20

EP 0 548 175 B1

2. Nukleotidsequenz nach Anspruch 1,
dadurch gekennzeichnet, daß der Promotor ein Promotor ist, der normalerweise mit einem Hitzeschockprotein beispielsweise von dem in Actinomyceten vorliegenden Typ assoziiert ist.

3. Nukleotidsequenz nach Anspruch 1 oder 2,
dadurch gekennzeichnet, daß das Motiv GCACTC 9N GAGTGC mindestens zum Teil innerhalb der Promotorsequenz liegt.

4. Nukleotidsequenz nach den Ansprüchen 2 und 3,
dadurch gekennzeichnet, daß der Promotor ausgewählt ist unter den folgenden Sequenzen:
P1 entsprechend einer der Sequenzen:

```
CATTGGCACTCCGCTTGACCGAGTGCTAATCGCGGTCATAGTCTCAGCTCTG
5'                                                    3'
```

oder

```
GCATTGGCACTCCGCTTGACCGAGTGCTAATCGCGGTCATAGTCTCAGCTCTG
5'                                                     3'
```

P2 entsprechend einer der Sequenzen:

```
GGAGGCCCCTAGCGCCTGCACTCTCCTACCCCGAGTGCTATTATTGGCGTTA
5'                                                    3'
```

oder

```
GGAGGCCCCTAGCGCCTGCACTCTCCTACCCCGAGTGCTAATTATTGGCGTTA
5'                                                     3'
```

5. Nukleotidsequenz nach Anspruch 1 oder 2,
dadurch gekennzeichnet, daß das Motiv GCACTC 9N GAGTGC von der Promotorsequenz getrennt ist.

6. Nukleotidsequenz nach Anspruch 5,
dadurch gekennzeichnet, daß das Motiv GCACTC 9N GAGTGC sich stromaufwärts von der Promotorsequenz, beispielsweise ungefähr 150 bis 200 Basen stromaufwärts, befindet.

7. Expressionsvektor,
der eine rekombinante Nukleotidsequenz nach einem der Ansprüche 1 bis 6 enthält und die Expression des Polypeptids ermöglichen kann.

8. Expressionsvektor nach Anspruch 7,
der ein Plasmid ist.

9. Mit dem Expressionsvektor nach einem der Ansprüche 7 und 8 transformierte Zelle,
die den Promotor erkennen und das Polypeptid exprimieren kann.

21

**10.** Zelle nach Anspruch 9,
die eine prokaryotische Zelle, beispielsweise aus der Gruppe der Actinomyceten, ist.

**11.** Verfahren zur Herstellung eines Polypeptids,
dadurch gekennzeichnet, daß es die folgenden Schritte umfaßt:
- eine Zelle mit einem Expressionsvektor nach einem der Ansprüche 7 oder 8 unter Bedingungen, die die Expression des Polypeptids erlauben, zu transformieren, wobei die Zelle in der Lage ist, den Promotor zu erkennen,
- das exprimierte Polypeptid zu gewinnen.

**12.** Verfahren nach Anspruch 11,
dadurch gekennzeichnet, daß die Bedingungen, die die Expression des Polypeptids erlauben, die Anwendung eines Hitzeschocks umfassen.

**13.** Verfahren nach einem der Ansprüche 11 oder 12,
dadurch gekennzeichnet, daß die Zelle eine Prokaryotische Zelle beispielsweise aus der Gruppe der Actinomyceten, und insbesondere aus der Gattung Streptomyces, ist.

**14.** Verfahren, um einem Promotor einen wärmeinduzierbaren Charakter zu vermitteln,
gekennzeichnet durch Juxtaposition einerseits einer Sequenz, die das Motiv GCACTC 9N GAGTGC enthält, und andererseits des Promotors, wobei die Sequenz, die das Motiv GCACTC 9N GAGTGC enthält, stromaufwärts vom Promotor angeordnet ist, oder durch Insertion der Sequenz, die das Motiv GCACTC 9N GAGTGC enthält, an einer Stelle, die mindestens teilweise innerhalb des Promotors liegt, wobei diese Stelle so ausgewählt wird, daß die einfache Insertion des Motivs die Aktivität des Promotors nicht stört.

**15.** Verfahren nach Anspruch 14,
dadurch gekennzeichnet, daß der Promotor ein in Actinomyceten, beispielsweise in Streptomyces oder Mycobacterium, vorliegender Promotor ist.

**16.** Regulationssequenz der Initiation der Transkription,
dadurch gekennzeichnet, daß sie einen Promotor in Verbindung mit dem Motiv GCACTC 9N GAGTGC trägt, wobei diese Sequenz keine kodierende Sequenz, die normalerweise mit diesem Promotor assoziiert ist, enthält.

**17.** Regulationssequenz der Initiation der Transkription,
dadurch gekennzeichnet, daß sie eine der folgenden Sequenzen trägt:
P1 entsprechend einer der Sequenzen:

**CATTGGCACTCCGCTTGACCGAGTGCTAATCGCGGTCATAGTCTCAGCTCTG**
**5'**                                                                                                    **3'**

oder

**GCATTGGCACTCCGCTTGACCGAGTGCTAATCGCGGTCATAGTCTCAGCTCTG**
**5'**                                                                                                    **3'**

P2 entsprechend einer der Sequenzen:

22

GGAGGCCCCTAGCGCCTGCACTCTCCTACCCCGAGTGCTATTATTGGCGTTA

5'                                                                                    3'

oder

GGAGGCCCCTAGCGCCTGCACTCTCCTACCCCGAGTGCTAATTATTGGCGTTA

5'                                                                                    3'

**18.** Hitzeschockpolypeptid, das trägt:
- entweder (i) eine der nachstehend angegebenen Aminosäuresequenzen,
- oder (ii) eine Aminosäuresequenz, die mindestens 90% Homologie zu dieser Sequenz aufweist,
- oder (iii) einen Teil der Sequenz (i) oder (ii), wobei dieser Teil den $NH_2$-terminalen Endabschnitt umfaßt und das Polypeptid eine Größe von ungefähr 18 kDa und einen isoelektrischen Punkt von ungefähr 9 hat,
- oder (iv) einen Teil der Sequenz (i), wobei dieser Teil den COOH-terminalen Bereich: Gly His Gly His Gly His Ser His enthält oder diesem Bereich entspricht,

```
1/1                                          31/11
ATG GCG AAG ATT CTG AAG TTC GAC GAG GAC GCC CGT CGC GCC CTT GAG CGC GGC GTG AAC
Met ala lys ile leu lys phe asp glu asp ala arg arg ala leu glu arg gly val asn
61/21                                        91/31
CAG CTG GCC GAC ACC GTC AAG GTG ACC ATC GGC CCC AAG GGC CGC AAC GTC GTC ATC GAC
gln leu ala asp thr val lys val thr ile gly pro lys gly arg asn val val ile asp
121/41                                       151/51
AAG AAG TTC GGC GCC CCG ACC ATC ACC AAC GAC GGC GTC ACC ATC GCC CGT GAG GTC GAG
lys lys phe gly ala pro thr ile thr asn asp gly val thr ile ala arg glu val glu
181/61                                       211/71
TGC GAC GAC CCG TAC GAG AAC CTC GGC GCC CAG CTC GTC AAG GAG GTG GCG ACC AAG ACC
cys asp asp pro tyr glu asn leu gly ala gln leu val lys glu val ala thr lys thr
241/81                                       271/91
AAC GAC ATC GCG GGT GAC GGC ACC ACC ACC GCG ACC GTG CTG GCC CAG GCG CTG GTC CGC
asn asp ile ala gly asp gly thr thr thr ala thr val leu ala gln ala leu val arg
301/101                                      331/111
GAG GGC CTG CGC AAC GTC GCC GCC GGC GCC TCC CCG GCC GCC CTG AAG AAG GGC ATC GAC
glu gly leu arg asn val ala ala gly ala ser pro ala ala leu lys lys gly ile asp
361/121                                      391/131
GCC GCC GTC GCC GCC GTC TCC GCC GAG CTG CTC GAC ACC GCG CGC CCG ATC GAC GAC AAG
ala ala val ala ala val ser ala glu leu leu asp thr ala arg pro ile asp asp lys
421/141                                      451/151
TCC GAC ATC GCC GCC GTC GCC GCG CTC TCC GCG CAG GAC AAG CAG GTC GGC GAG CTC ATC
ser asp ile ala ala val ala ala leu ser ala gln asp lys gln val gly glu leu ile
481/161                                      511/171
GCC CAG GCG ATG GAC AAG GTC GGC AAG GAC GGT GTC ATC ACC GTC GAG GAG TCC AAC ACC
ala gln ala met asp lys val gly lys asp gly val ile thr val glu glu ser asn thr
541/181                                      571/191
TTC GGT GTC GAC CTG GAC TTC ACC GAG GGC ATG GCC TTC GAC AAG GGC TAC CTG TCC CCG
phe gly val asp leu asp phe thr glu gly met ala phe asp lys gly tyr leu ser pro
601/201                                      631/211
TAC ATG GTG ACC GAC CAG GAG CGT ATG GAG GCC GTC CTC GAC GAC CCG TAC ATC CTG ATC
tyr met val thr asp gln glu arg met glu ala val leu asp asp pro tyr ile leu ile
661/221                                      691/231
CAC CAG GGC AAG ATC GGT TCG ATC CAG GAC CTG CTG CCG CTG CTG GAG AAG GTC ATC CAG
his gln gly lys ile gly ser ile gln asp leu leu pro leu leu glu lys val ile gln
721/241                                      751/251
GCG GGT GGC TCC AAG CCG CTG CTG ATC ATC GCC GAG GAC GTC GAG GGC GAG GCC CTG TCG
ala gly gly ser lys pro leu leu ile ile ala glu asp val glu gly glu ala leu ser
781/261                                      811/271
ACC CTG GTG GTC AAC AAG ATC CGC GGC ACG TTC AAC GCC GTC GCC GTC AAG GCG CCC GGC
thr leu val val asn lys ile arg gly thr phe asn ala val ala val lys ala pro gly
841/281                                      871/291
TTC GGT GAC CGC CGC AAG GCG ATG CTC GGC GAC ATG GCC ACC CTC ACC GGT GCC ACC GTC
phe gly asp arg arg lys ala met leu gly asp met ala thr leu thr gly ala thr val
901/301                                      931/311
ATC GCC GAG GAG GTC GGC CTC AAG CTC GAC CAG GCC GGT CTG GAC GTG CTG GGC ACC GCC
ile ala glu glu val gly leu lys leu asp gln ala gly leu asp val leu gly thr ala
961/321                                      991/331
CGC CGC GTC ACC GTC ACC AAG GAC GAC ACG ACC ATC GTG GAC GGC GGC GGC AAC GCC GAG
arg arg val thr val thr lys asp asp thr thr ile val asp gly gly gly asn ala glu
1021/341                                     1051/351
GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp
1081/361                                     1111/371
GAC CGC GAG AAG CTC CAG GAG CGC CTC GCC AAG CTG GCC GGC GGC GTC TGC GTG ATC CGC
asp arg glu lys leu gln glu arg leu ala lys leu ala gly gly val cys val ile arg
1141/381                                     1171/391
GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile
```

```
1201/401                                    1231/411
TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val
1261/421                                    1291/431
CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val
```

oder die Sequenz:

```
1/1                                         31/11
ATG GCG AAG ATT CTG AAG TTC GAC GAG GAC GCC CGT CGC GCC CTT GAG CGC GGC GTG AAC
Met ala lys ile leu lys phe asp glu asp ala arg arg ala leu glu arg gly val asn
61/21                                       91/31
CAG CTG GCC GAC ACC GTC AAG GTG ACC ATC GGC CCC AAG GGC CGC AAC GTC GTC ATC GAC
gln leu ala asp thr val lys val thr ile gly pro lys gly arg asn val val ile asp
121/41                                      151/51
AAG AAG TTC GGC GCC CCG ACC ATC ACC AAC GAC GGC GTC ACC ATC GCC CGT GAG GTC GAG
lys lys phe gly ala pro thr ile thr asn asp gly val thr ile ala arg glu val glu
181/61                                      211/71
TGC GAC GAC CCG TAC GAG AAC CTC GGC GCC CAG CTC GTC AAG GAG GTG GCG ACC AAG ACC
cys asp asp pro tyr glu asn leu gly ala gln leu val lys glu val ala thr lys thr
241/81                                      271/91
AAC GAC ATC GCG GGT GAC GGC ACC ACC ACC GCC ACC GTG CTG GCC CAG GCG CTG GTC CGC
asn asp ile ala gly asp gly thr thr thr ala thr val leu ala gln ala leu val arg
301/101                                     331/111
GAG GGC CTG CGC AAC GTC GCC GCC GGC GCC TCC CCG GCC GCC CTG AAG AAG GGC ATC GAC
glu gly leu arg asn val ala ala gly ala ser pro ala ala leu lys lys gly ile asp
361/121                                     391/131
GCC GCG GTC GCC GCC GTC TCC GCC GAG CTG CTC GAC ACC GCG CGC CCG ATC GAC GAC AAG
ala ala val ala ala val ser ala glu leu leu asp thr ala arg pro ile asp asp lys
421/141                                     451/151
TCC GAC ATC GCC GCC GTC GCC GCG CTG TCC GCG CAG GAC AAG CAG GTC GGC GAG CTC ATC
ser asp ile ala ala val ala ala leu ser ala gln asp lys gln val gly glu leu ile
481/161                                     511/171
GCC GAG GCG ATG GAC AAG GTC GGC AAG GAC GGT GTC ATC ACC GTC GAG GAG TCC AAC ACC
ala glu ala met asp lys val gly lys asp gly val ile thr val glu glu ser asn thr
541/181                                     571/191
TTC GGT GTC GAC CTG GAC TTC ACC GAG GGC ATG GCC TTC GAC AAG GGC TAC CTG TCC CCG
phe gly val asp leu asp phe thr glu gly met ala phe asp lys gly tyr leu ser pro
601/201                                     631/211
TAC ATC GTG ACC GAC CAG GAG CGT ATG GAG GCC GTC CTC GAC GAC CCG TAC ATC CTG ATC
tyr met val thr asp gln glu arg met glu ala val leu asp asp pro tyr ile leu ile
661/221                                     691/231
CAC CAG GGC AAG ATC GGT TCG ATC CAG GAC CTG CTG CCG CTG CTG GAG AAG GTC ATC CAG
his gln gly lys ile gly ser ile gln asp leu leu pro leu leu glu lys val ile gln
721/241                                     751/251
GCG GGT GGC TCC AAG CCG CTG CTC ATC ATC GCC GAG GAC GTC GAG GGC GAG GCC CTG TCG
ala gly gly ser lys pro leu leu ile ile ala glu asp val glu gly glu ala leu ser
781/261                                     811/271
ACC CTC GTG GTC AAC AAG ATC CGC GGC ACG TTC AAC GCC GTC GCC GTC AAG GCG CCC GGC
thr leu val val asn lys ile arg gly thr phe asn ala val ala val lys ala pro gly
```

25

841/281                                              871/291
TTC GGT GAC CGC CGC AAG GCG ATG CTC GGC GAC ATG GCC ACC CTC ACC GGT GCC ACC GTC
phe gly asp arg arg lys ala met leu gly asp met ala thr leu thr gly ala thr val
901/301                                              931/311
ATC GCC CAG GAG GTC GGC CTC AAG CTC GAC CAG GCC GGT CTG GAC GTG CTG GGC ACC GCC
ile ala glu glu val gly leu lys leu asp gln ala gly leu asp val leu gly thr ala
961/321                                              991/331
CGC CGC GTC ACC GTC ACC AAG GAC GAC ACG ACC ATC GTG GAC GGC GGC GGC AAC GCC GAG
arg arg val thr val thr lys asp asp thr thr ile val asp gly gly gly asn ala glu

1021/341                                             1051/351
GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp
1081/361                                             1111/371
GAC CGC GAG AAG CTC CAG GAG CGC CTC GCC AAG CTG GCC GGC CGC GTC TGC GTG ATC CGC
asp arg glu lys leu gln glu arg leu ala lys leu ala gly gly val cys val ile arg
1141/381                                             1171/391
GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile
1201/401                                             1231/411
TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val
1261/421                                             1291/431
CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val
1321/441                                             1351/451
GCG GTC GTC CGC CGC GCC GCC GTC GAG CCG CTG CGC TGG ATC GCC GAG AAC GCC GGC CTC
ala val val arg arg ala ala val glu pro leu arg trp ile ala glu asn ala gly leu
1381/461                                             1411/471
GAG GGC TAC GTC ATC ACC ACC AAG GTG GCG GAG CTC GAC AAG GGC CAG GGC TTC AAC GCG
glu gly tyr val ile thr thr lys val ala glu leu asp lys gly gln gly phe asn ala
1441/481                                             1471/491
GCC ACC GGC GAG TAC GGC GAC CTG GTC AAG GCC GGC GTC ATC GAC CCG GTC AAG GTC ACC
ala thr gly glu tyr gly asp leu val lys ala gly val ile asp pro val lys val thr
1501/501                                             1531/511
CGC TCC GCC CTG GAG AAC GCG GCC TCC ATC GCC TCC CTG CTC CTG ACG ACC GAG ACC CTG
arg ser ala leu glu asn ala ala ser ile ala ser leu leu leu thr thr glu thr leu
1561/521                                             1591/531
GTC GTC GAG AAG CCG GCC GAG GAG GAG CCC GAG GCC GGT CAC GGT CAC GGG CAC AGC CAC
val val glu lys pro ala glu glu glu pro glu ala gly his gly his gly his ser his

19. Polypeptid nach Anspruch 18,

gekennzeichnet durch eine Aminosäuresequenz, die sich von der mit 1 bezeichneten Aminosäure höchstens bis zur Aminosäure 170 erstreckt.

20. Für ein Hitzeschockprotein nach einem der Ansprüche 18 oder 19 kodierende Nukleinsäuresequenz.

21. Nukleinsäuresequenz nach Anspruch 20,
dadurch gekennzeichnet, daß sie mindestens ein Fragment der in Anspruch 18 angegebenen Sequenz enthält.

26

FIGURE 1

FIGURE 2

EP 0 548 175 B1

P1 :

5' CATTG[GCACTC]CGCTTGACC[GAGTGC]TAATCGCGGTCATAGTCTCAGCTCTG 3'

$-30$

5' GCATTG[GCACTC]CGCTTGACC[GAGTGC]TAATCGCGGTCATAGTCTCAGCTCTG 3'
$-10$ $+1$

P2 :

5' GGAGGCCCCTAGCGCCT[GCACTC]TCCTACCCC[GAGTGC]TATTATTGGCGTTA 3'

$-30$

5' GGAGGCCCCTAGCGCCT[GCACTC]TCCTACCCC[GAGTGC]TAATTATTGGCGTTA 3'
$-10$ $+1$

Motif consensuel : GCACTC 7N CCGAGTGCTAAT

Motif consensuel palindromique : [GCACTC] 9N [GAGTGC]

FIGURE 3

FIGURE 4

FIGURE 5

EP 0 548 175 B1

```
1/1                                       31/11
GTG ACG ACC GCC AGC TCC AAG GTT GCC ATC AAG CCG CTC GAG GAC CGC ATC GTG GTC CAG
val thr thr ala ser ser lys val ala ile lys pro leu glu asp arg ile val val gln
61/21                                     91/31
CCG CTC GAC GCC GAG CAG ACC ACG GCT TCG GGC CTG GTC ATC CCG GAC ACC GCG AAG GAG
pro leu asp ala glu gln thr thr ala ser gly leu val ile pro asp thr ala lys glu
121/41                                    151/51
AAG CCC CAG GAG GGC GTC GTC CTC GCG GTC GGC CCG GGC CGC TTC GAG AAC GGC GAG CGC
lys pro gln glu gly val val leu ala val gly pro gly arg phe glu asn gly glu arg
181/61                                    211/71
CTG CCG CTC GAC GTC AAG ACC GGC GAC GTC GTG CTG TAC AGC AAG TAC GGC GGC ACC GAG
leu pro leu asp val lys thr gly asp val val leu tyr ser lys tyr gly gly thr glu
241/81                                    271/91
GTC AAG TAC AAC GGC GAG GAG TAC CTC GTC CTC TCG GCC CGC GAC GTT CTC GCC ATC ATC
val lys tyr asn gly glu glu tyr leu val leu ser ala arg asp val leu ala ile ile
301/101
GAG AAG TAG
glu lys AMB
```

## FIGURE 6



ATG GCG AAG ATT CTG AAG TTC GAC GAG GAC GCC CGT CGC GCC CTT GAG CGC GGC GTG AAC
Met ala lys ile leu lys phe asp glu asp ala arg arg ala leu glu arg gly val asn

CAG CTG GCC GAC ACC GTC AAG GTG ACC ATC GGC CCC AAG GGC CGC AAC GTC GTC ATC GAC
gln leu ala asp thr val lys val thr ile gly pro lys gly arg asn val val ile asp

AAG AAG TTC GGC GCC CCG ACC ATC ACC AAC GAC GGC GTC ACC ATC GCC CGT GAG GTC GAG
lys lys phe gly ala pro thr ile thr asn asp gly val thr ile ala arg glu val glu

TGC GAC GAC CCG TAC GAG AAC CTC GGC GCC CAG CTC GTC AAG GAG GTG GCG ACC AAG ACC
cys asp asp pro tyr glu asn leu gly ala gln leu val lys glu val ala thr lys thr

AAC GAC ATC GCG GGT GAC GGC ACC ACC ACC GCG ACC GTG CTG GCC CAG GCG CTG GTC CGC
asn asp ile ala gly asp gly thr thr thr ala thr val leu ala gln ala leu val arg

GAG GGC CTG CGC AAC GTC GCC GCC GGC GCC TCC CCG GCC GCC CTG AAG AAG GGC ATC GAC
glu gly leu arg asn val ala ala gly ala ser pro ala ala leu lys lys gly ile asp

GCC GCC GTC GCC GCC GTC TCC GCC GAG CTG CTC GAC ACC GCG CGC CCG ATC GAC GAC AAG
ala ala val ala ala val ser ala glu leu leu asp thr ala arg pro ile asp asp lys

TCC GAC ATC GCC GCC GTC GCC GCG CTC TCC GCG CAG GAC AAG CAG GTC GGC GAG CTC ATC
ser asp ile ala ala val ala ala leu ser ala gln asp lys gln val gly glu leu ile

FIGURE 6 (suite)

GCC GAG GCG ATG GAC AAG GTC GGC AAG GAC GGT GTC ATC ACC GTC GAG GAG TCC AAC ACC
ala glu ala met asp lys val gly lys asp gly val ile thr val glu glu ser asn thr

TTC GGT GTC GAC CTG GAC TTC ACC GAG GGC ATG GCC TTC GAC AAG GGC TAC CTG TCC CCG
phe gly val asp leu asp phe thr glu gly met ala phe asp lys gly tyr leu ser pro

TAC ATG GTG ACC GAC CAG GAG CGT ATG GAG GCC GTC CTC GAC GAC CCG TAC ATC CTG ATC
tyr met val thr asp gln glu arg met glu ala val leu asp asp pro tyr ile leu ile

CAC CAG GGC AAG ATC GGT TCG ATC CAG GAC CTG CTG CCG CTG CTG GAG AAG GTC ATC CAG
his gln gly lys ile gly ser ile gln asp leu leu pro leu leu glu lys val ile gln

GCG GGT GGC TCC AAG CCG CTG CTG ATC ATC GCC GAG GAC GTC GAG GGC GAG GCC CTG TCG
ala gly gly ser lys pro leu leu ile ile ala glu asp val glu gly glu ala leu ser

ACC CTG GTG GTC AAC AAG ATC CGC GGC ACG TTC AAC GCC GTC GCC GTC AAG GCG CCC GGC
thr leu val val asn lys ile arg gly thr phe asn ala val ala val lys ala pro gly

TTC GGT GAC CGC CGC AAG GCG ATG CTC GGC GAC ATG GCC ACC CTC ACC GGT GCC ACC GTC
phe gly asp arg arg lys ala met leu gly asp met ala thr leu thr gly ala thr val

ATC GCC GAG GAG GTC GGC CTC AAG CTC GAC CAG GCC GGT CTG GAC GTG CTG GGC ACC GCC
ile ala glu glu val gly leu lys leu asp gln ala gly leu asp val leu gly thr ala

EP 0 548 175 B1

FIGURE 6   (fin)

961/321                                         991/331
CGC CGC GTC ACC GTC ACC AAG GAC GAC ACG ACC ATC GTG GAC GGC GGC GGC AAC GCC GAG
arg arg val thr val thr lys asp asp thr thr ile val asp gly gly gly asn ala glu
1021/341                                        1051/351
GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp

1081/361                                        1111/371
GAC CGC GAG AAG CTC CAG GAG CGC CTC GCC AAG CTG GCC GGC GGC GTC TGC GTG ATC CGC
asp arg glu lys leu gln glu arg leu ala lys leu ala gly gly val cys val ile arg
1141/381                                        1171/391
GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile
1201/401                                        1231/411
TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val
1261/421                                        1291/431
CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val

EP 0 548 175 B1

# FIGURE 7

```
            |   10     |   20      |   30       |   40  .    |   50      |   60
    1  CCGGCCGGGC  TGAGGTTGGC  TGGCTGGCCG  GGTTCGGCCG  GTGGGTCGAG  GTGGCCTGGC   60
   61  CGGGCTCGCC  AGGGTGAGTT  GGCCGAGCCG  AGGCGGCCCC  GGGGCTCCCC  GGGCCGAGTT  120
  121  GGCGCGGCCA  GGCCAGGGCT  CAGCAGGGTG  GGGGAGTGGG  GCAGGCGGCC  CGGTAGGGGA  180
  181  GTGCGGGAGG  GCAGCGCGCG  CCGCGCGCAT  TGGCACTCCG  CTTGACCGAG  TGCTAATCGC  240
  241  GGTCATAGTC  TCAGCTCTGG  CACTCCCCGC  AGGAGAGTGC  CAACACAGCG  ACGGGCAGGT  300
  301  CCGGCACCCG  CGACGACGGA  TCGACCTGGT  CGCCACACTC  AGATCAGTTA  ACCCCGTGAT  360
  361  CTCCGAAGGG  GGAGGTCGGA  TCGTGACGAC  CGCCAGCTCC  AAGGTTGCCA  TCAAGCCGCT  420
  421  CGAGGACCGC  ATCGTGGTCC  AGCCGCTCGA  CGCCGAGCAG  ACCACGGCTT  CGGGCCTGGT  480
  481  CATCCCGGAC  ACCGCGAAGG  AGAAGCCCCA  GGAGGGCGTC  GTCCTCGCGG  TCGGCCCGGG  540
  541  CCGCTTCGAG  AACGGCGAGC  GCCTGCCGCT  CGACGTCAAG  ACCGGCGACG  TCGTGCTGTA  600
  601  CAGCAAGTAC  GGCGGCACCG  AGGTCAAGTA  CAACGGCGAG  GAGTACCTCG  TCCTCTCGGC  660
  661  CCGCGACGTT  CTCGCCATCA  TCGAGAAGTA  GCAGGCCGGA  GCGGTCCGGG  CGCGAGCCCG  720
  721  GACGGCAGAC  TCCACCTTTT  TCCTGAAGCG  CGCCCCTGGC  CCCCGCGAGT  GTTTGCCGGG  780
  781  TGGCGAGGGG  CGCGTTTCAT  TTCGAGAGCG  CGGCGGCAGG  CCGCTCCGAG  AGGATTCGAA  840
  841  AAGCTCCCAT  GGCGAAGATT  CTGAAGTTCG  ACGAGGACGC  CCGTCGCGCC  CTTGAGCGCG  900
  901  GCGTGAACCA  GCTGGCCGAC  ACCGTCAAGG  TGACCATCGG  CCCCAAGGGC  CGCAACGTCG  960
  961  TCATCGACAA  GAAGTTCGGC  GCCCCGACCA  TCACCAACGA  CGGCGTCACC  ATCGCCCGTG  1020
 1021  AGGTCGAGTG  CGACGACCCG  TACGAGAACC  TCGGCGCCCA  GCTCGTCAAG  GAGGTGGCGA  1080
 1081  CCAAGACCAA  CGACATCGCG  GGTGACGGCA  CCACCACCGC  GACCGTGCTG  GCCCAGGCGC  1140
 1141  TGGTCCGCGA  GGGCCTGCGC  AACGTCGCCG  CCGGCGCCTC  CCCGGCCGCC  CTGAAGAAGG  1200
 1201  GCATCGACGC  CGCCGTCGCC  GCCGTCTCCG  CCGAGCTGCT  CGACACCGCG  CGCCCGATCG  1260
 1261  ACGACAAGTC  CGACATCGCC  GCCGTCGCCG  CGCTCTCCGC  GCAGGACAAG  CAGGTCGGCG  1320
 1321  AGCTCATCGC  CGAGGCGATG  GACAAGGTCG  GCAAGGACGG  TGTCATCACC  GTCGAGGAGT  1380
```

EP 0 548 175 B1

FIGURE 7 (fin)

```
1381 CCAACACCTT CGGTGTCGAC CTGGACTTCA  CCGAGGGCAT GGCCTTCGAC AAGGGCTACC 1440
1441 TGTCCCCGTA CATGGTGACC GACCAGGAGC  GTATGGAGGC CGTCCTCGAC GACCCGTACA 1500
1501 TCCTGATCCA CCAGGGCAAG ATCGGTTCGA  TCCAGGACCT GCTGCCGCTG CTGGAGAAGG 1560
1561 TCATCCAGGC GGGTGGCTCC AAGCCGCTGC  TGATCATCGC CGAGGACGTC GAGGGCGAGG 1620
1621 CCCTGTCGAC CCTGGTGGTC AACAAGATCC  GCGGCACGTT CAACGCCGTC GCCGTCAAGG 1680
1681 CGCCCGGCTT CGGTGACCGC CGCAAGGCGA  TGCTCGGCGA CATGGCCACC CTCACCGGTG 1740
1741 CCACCGTCAT CGCCGAGGAG GTCGGCCTCA  AGCTCGACCA GGCCGGTCTG GACGTGCTGG 1800
1801 GCACCGCCCG CCGCGTCACC GTCACCAAGG  ACGACACGAC CATCGTGGAC GGCGGCGGCA 1860
1861 ACGCCGAGGA CGTCCAGGGC CGCGTCGCCC  AGATCAAGGC CGAGATCGAG TCGACCGACT 1920
1921 CGGACTGGGA CCGCGAGAAG CTCCAGGAGC  GCCTCGCCAA GCTGGCCGGC GGCGTCTGCG 1980
1981 TGATCCGCGT CGGCGCGGCC ACCGAGGTCG  AGCTGAAGGA GCGCAAGCAC CGTCTGGAGG 2040
2041 ACGCCATCTC CGCGACCCGC GCCGCGGTCG  AGGAGGGCAT CGTCTCCGGT GGTGGCTCCG 2100
2101 CGCTGGTCCA CGCCGTCAAG GTCCTGGACG  ACAACCTCGG CCGCACCGGC GACGAGGCCA 2160
2161 CCGGTGT                                                        2167
        |   10    |   20    |   30      |   40    |   50    |   60
```

## FIGURE 8

ATG GCG AAG ATT CTG AAG TTC GAC GAG GAC GCC CGT CGC GCC CTT GAG CGC GGC GTG AAC
Met ala lys ile leu lys phe asp glu asp ala arg arg ala leu glu arg gly val asn

CAG CTG GCC GAC ACC GTC AAG GTG ACC ATC GGC CCC AAG GGC CGC AAC GTC GTC ATC GAC
gln leu ala asp thr val lys val thr ile gly pro lys gly arg asn val val ile asp

AAG AAG TTC GGC GCC CCG ACC ATC ACC AAC GAC GGC GTC ACC ATC GCC CGT GAG GTC GAG
lys lys phe gly ala pro thr ile thr asn asp gly val thr ile ala arg glu val glu

TGC GAC GAC CCG TAC GAG AAC CTC GGC GCC CAG CTC GTC AAG GAG GTG GCG ACC AAG ACC
cys asp asp pro tyr glu asn leu gly ala gln leu val lys glu val ala thr lys thr

AAC GAC ATC GCG GGT GAC GGC ACC ACC ACC GCG ACC GTG CTG GCC CAG GCG CTG GTC CGC
asn asp ile ala gly asp gly thr thr thr ala thr val leu ala gln ala leu val arg

GAG GGC CTG CGC AAC GTC GCC GCC GGC GCC TCC CCG GCC GCC CTG AAG AAG GGC ATC GAC
glu gly leu arg asn val ala ala gly ala ser pro ala ala leu lys lys gly ile asp

GCC GCC GTC GCC GCC GTC TCC GCC GAG CTG CTC GAC ACC GCG CGC CCG ATC GAC GAC AAG
ala ala val ala ala val ser ala glu leu leu asp thr ala arg pro ile asp asp lys

TCC GAC ATC GCC GCC GTC GCC GCG CTC TCC GCG CAG GAC AAG CAG GTC GGC GAG CTC ATC
ser asp ile ala ala val ala ala leu ser ala gln asp lys gln val gly glu leu ile

EP 0 548 175 B1

FIGURE 8 (suite a)

481/161                                             511/171
GCC GAG GCG ATG GAC AAG GTC GGC AAG GAC GGT GTC ATC ACC GTC GAG GAG TCC AAC ACC
ala glu ala met asp lys val gly lys asp gly val ile thr val glu glu ser asn thr
541/181                                             571/191
TTC GGT GTC GAC CTG GAC TTC ACC GAG GGC ATG GCC TTC GAC AAG GGC TAC CTG TCC CCG
phe gly val asp leu asp phe thr glu gly met ala phe asp lys gly tyr leu ser pro
601/201                                             631/211
TAC ATG GTG ACC GAC CAG GAG CGT ATG GAG GCC GTC CTC GAC GAC CCG TAC ATC CTG ATC
tyr met val thr asp gln glu arg met glu ala val leu asp asp pro tyr ile leu ile
661/221                                             691/231
CAC CAG GGC AAG ATC GGT TCG ATC CAG GAC CTG CTG CCG CTG CTG GAG AAG GTC ATC CAG
his gln gly lys ile gly ser ile gln asp leu leu pro leu leu glu lys val ile gln
721/241                                             751/251
GCG GGT GGC TCC AAG CCG CTG CTG ATC ATC GCC GAG GAC GTC GAG GGC GAG GCC CTG TCG
ala gly gly ser lys pro leu leu ile ile ala glu asp val glu gly glu ala leu ser
781/261                                             811/271
ACC CTG GTG GTC AAC AAG ATC CGC GGC ACG TTC AAC GCC GTC GCC GTC AAG GCG CCC GGC
thr leu val val asn lys ile arg gly thr phe asn ala val ala val lys ala pro gly
841/281                                             871/291
TTC GGT GAC CGC CGC AAG GCG ATG CTC GGC GAC ATG GCC ACC CTC ACC GGT GCC ACC GTC
phe gly asp arg arg lys ala met leu gly asp met ala thr leu thr gly ala thr val
901/301                                             931/311
ATC GCC GAG GAG GTC GGC CTC AAG CTC GAC CAG GCC GGT CTG GAC GTG CTG GGC ACC GCC
ile ala glu glu val gly leu lys leu asp gln ala gly leu asp val leu gly thr ala

FIGURE 8 (suite b)

961/321                                                    991/331
CGC CGC GTC ACC GTC ACC AAG GAC GAC ACG ACC ATC GTG GAC GGC GGC GGC AAC GCC GAG
arg arg val thr val thr lys asp asp thr thr ile val asp gly gly gly asn ala glu
1021/341                                                   1051/351
GAC GTC CAG GGC CGC GTC GCC CAG ATC AAG GCC GAG ATC GAG TCG ACC GAC TCG GAC TGG
asp val gln gly arg val ala gln ile lys ala glu ile glu ser thr asp ser asp trp
1081/361                                                   1111/371
GAC CGC GAG AAG CTC CAG GAG CGC CTC GCC AAG CTG GCC GGC GGC GTC TGC GTG ATC CGC
asp arg glu lys leu gln glu arg leu ala lys leu ala gly gly val cys val ile arg
1141/381                                                   1171/391
GTC GGC GCG GCC ACC GAG GTC GAG CTG AAG GAG CGC AAG CAC CGT CTG GAG GAC GCC ATC
val gly ala ala thr glu val glu leu lys glu arg lys his arg leu glu asp ala ile
1201/401                                                   1231/411
TCC GCG ACC CGC GCC GCG GTC GAG GAG GGC ATC GTC TCC GGT GGT GGC TCC GCG CTG GTC
ser ala thr arg ala ala val glu glu gly ile val ser gly gly gly ser ala leu val
1261/421                                                   1291/431
CAC GCC GTC AAG GTC CTG GAC GAC AAC CTC GGC CGC ACC GGC GAC GAG GCC ACC GGT GTC
his ala val lys val leu asp asp asn leu gly arg thr gly asp glu ala thr gly val
1321/441                                                   1351/451
GCG GTC GTC CGC CGC GCC GCC GTC GAG CCG CTG CGC TGG ATC GCC GAG AAC GCC GGC CTC
ala val val arg arg ala ala val glu pro leu arg trp ile ala glu asn ala gly leu
1381/461                                                   1411/471
GAG GGC TAC GTC ATC ACC ACC AAG GTG GCG GAG CTC GAC AAG GGC CAG GGC TTC AAC GCG
glu gly tyr val ile thr thr lys val ala glu leu asp lys gly gln gly phe asn ala

EP 0 548 175 B1

FIGURE 8 (fin)

1441/481                                        1471/491
GCC ACC GGC GAG TAC GGC GAC CTG GTC AAG GCC GGC GTC ATC GAC CCG GTC AAG GTC ACC
ala thr gly glu tyr gly asp leu val lys ala gly val ile asp pro val lys val thr
1501/501                                        1531/511
CGC TCC GCC CTG GAG AAC GCG GCC TCC ATC GCC TCC CTG CTC CTG ACG ACC GAG ACC CTG
arg ser ala leu glu asn ala ala ser ile ala ser leu leu leu thr thr glu thr leu
1561/521                                        1591/531
GTC GTC GAG AAG CCG GCC GAG GAG GAG CCC GAG GCC GGT CAC GGT CAC GGG CAC AGC CAC
val val glu lys pro ala glu glu glu pro glu ala gly his gly his gly his ser his

EP 0 548 175 B1

FIGURE 9

```
         |   10      |   20      |   30        |   40      |   50      |   60
    1  CCGGCCGGGC TGAGGTTGGC TGGCTGGCCG   GGTTCGGCCG GTGGGTCGAG GTGGCCTGGC   60
   61  CGGGCTCGCC AGGGTGAGTT GGCCGAGCCG   AGGCGGCCCC GGGGCTCCCC GGGCCGAGTT  120
  121  GGCGCGGCCA GGCCAGGGCT CAGCAGGGTG   GGGGAGTGGG GCAGGCGGCC CGGTAGGGGA  180
  181  GTGCGGGAGG GCAGCGCGCG CCGCGCGCAT   TGGCACTCCG CTTGACCGAG TGCTAATCGC  240
  241  GGTCATAGTC TCAGCTCTGG CACTCCCCGC   AGGAGAGTGC CAACACAGCG ACGGGCAGGT  300
  301  CCGGCACCCG CGACGACGGA TCGACCTGGT   CGCCACACTC AGATCAGTTA ACCCCGTGAT  360
  361  CTCCGAAGGG GGAGGTCGGA TCGTGACGAC   CGCCAGCTCC AAGGTTGCCA TCAAGCCGCT  420
  421  CGAGGACCGC ATCGTGGTCC AGCCGCTCGA   CGCCGAGCAG ACCACGGCTT CGGGCCTGGT  480
  481  CATCCCGGAC ACCGCGAAGG AGAAGCCCCA   GGAGGGCGTC GTCCTCGCGG TCGGCCCGGG  540
  541  CCGCTTCGAG AACGGCGAGC GCCTGCCGCT   CGACGTCAAG ACCGGCGACG TCGTGCTGTA  600
  601  CAGCAAGTAC GGCGGCACCG AGGTCAAGTA   CAACGGCGAG GAGTACCTCG TCCTCTCGGC  660
  661  CCGCGACGTT CTCGCCATCA TCGAGAAGTA   GCAGGCCGGA GCGGTCCGGG CGCGAGCCCG  720
  721  GACGGCAGAC TCCACCTTTT TCCTGAAGCG   CGCCCCTGGC CCCCGCGAGT GTTTGCCGGG  780
  781  TGGCGAGGGG CGCGTTTCAT TTCGAGAGCG   CGGCGGCAGG CCGCTCCGAG AGGATTCGAA  840
  841  AAGCTCCCAT GGCGAAGATT CTGAAGTTCG   ACGAGGACGC CCGTCGCGCC CTTGAGCGCG  900
  901  GCGTGAACCA GCTGGCCGAC ACCGTCAAGG   TGACCATCGG CCCCAAGGGC CGCAACGTCG  960
  961  TCATCGACAA GAAGTTCGGC GCCCCGACCA   TCACCAACGA CGGCGTCACC ATCGCCCGTG 1020
 1021  AGGTCGAGTG CGACGACCCG TACGAGAACC   TCGGCGCCCA GCTCGTCAAG GAGGTGGCGA 1080
 1081  CCAAGACCAA CGACATCGCG GGTGACGGCA   CCACCACCGC GACCGTGCTG GCCCAGGCGC 1140
 1141  TGGTCCGCGA GGGCCTGCGC AACGTCGCCG   CCGGCGCCTC CCCGGCCGCC CTGAAGAAGG 1200
 1201  GCATCGACGC CGCCGTCGCC GCCGTCTCCG   CCGAGCTGCT CGACACCGCG CGCCCGATCG 1260
 1261  ACGACAAGTC CGACATCGCC GCCGTCGCCG   CGCTCTCCGC GCAGGACAAG CAGGTCGGCG 1320
 1321  AGCTCATCGC CGAGGCGATG GACAAGGTCG   GCAAGGACGG TGTCATCACC GTCGAGGAGT 1380
```

EP 0 548 175 B1

FIGURE 9 (fin)

```
1381 CCAACACCTT CGGTGTCGAC CTGGACTTCA   CCGAGGGCAT GGCCTTCGAC AAGGGCTACC 1440
1441 TGTCCCCGTA CATGGTGACC GACCAGGAGC   GTATGGAGGC CGTCCTCGAC GACCCGTACA 1500
1501 TCCTGATCCA CCAGGGCAAG ATCGGTTCGA   TCCAGGACCT GCTGCCGCTG CTGGAGAAGG 1560
1561 TCATCCAGGC GGGTGGCTCC AAGCCGCTGC   TGATCATCGC CGAGGACGTC GAGGGCGAGG 1620
1621 CCCTGTCGAC CCTGGTGGTC AACAAGATCC   GCGGCACGTT CAACGCCGTC GCCGTCAAGG 1680
1681 CGCCCGGCTT CGGTGACCGC CGCAAGGCGA   TGCTCGGCGA CATGGCCACC CTCACCGGTG 1740
1741 CCACCGTCAT CGCCGAGGAG GTCGGCCTCA   AGCTCGACCA GGCCGGTCTG GACGTGCTGG 1800
1801 GCACCGCCCG CCGCGTCACC GTCACCAAGG   ACGACACGAC CATCGTGGAC GGCGGCGGCA 1860
1861 ACGCCGAGGA CGTCCAGGGC CGCGTCGCCC   AGATCAAGGC CGAGATCGAG TCGACCGACT 1920
1921 CGGACTGGGA CCGCGAGAAG CTCCAGGAGC   GCCTCGCCAA GCTGGCCGGC GGCGTCTGCG 1980
1981 TGATCCGCGT CGGCGCGGCC ACCGAGGTCG   AGCTGAAGGA GCGCAAGCAC CGTCTGGAGG 2040
2041 ACGCCATCTC CGCGACCCGC GCCGCGGTCG   AGGAGGGCAT CGTCTCCGGT GGTGGCTCCG 2100
2101 CGCTGGTCCA CGCCGTCAAG GTCCTGGACG   ACAACCTCGG CCGCACCGGC GACGAGGCCA 2160
2161 CCGGTGTCGC GGTCGTCCGC CGCGCCGCCG   TCGAGCCGCT GCGCTGGATC GCCGAGAACG 2220
2221 CCGGCCTCGA GGGCTACGTC ATCACCACCA   AGGTGGCGGA GCTCGACAAG GGCCAGGGCT 2280
2281 TCAACGCGGC CACCGGCGAG TACGGCGACC   TGGTCAAGGC CGGCGTCATC GACCCGGTCA 2340
2341 AGGTCACCCG CTCCGCCCTG GAGAACGCGG   CCTCCATCGC CTCCCTGCTC CTGACGACCG 2400
2401 AGACCCTGGT CGTCGAGAAG CCGGCCGAGG   AGGAGCCCGA GGCCGGTCAC GGTCACGGGC 2460
2461 ACAGCCACTG AGGCTGACCC CTTCCGCAGC   CGAGGCCCGG CTCCCCGTCG CGGGGAGCCG 2520
2521 GGCCTCCGGC GTGTCCGGGA CCCCCCGGGA   CGCGCGACGC CTACCGCGGC CCGTACTTGC 2580
2581 GGCCGGTACG CGAGGTCATC CCGGTCAGCA   GGGCCCGCGG GGTCAGCTTC ACCAGGCCCA 2640
2641 TCAGCGCCTT GTACCGAGGG TCCGGGAT                                       2668
        |  10      |  20      |  30        |  40      |  50      |  60
```